# NEW EUROPEAN PATENT SPECIFICATION

(11) **EP 3 315 145 B2**
(45) Date of publication and mention of the opposition decision: **16.07.2025**
(45) Mention of the grant of the patent: 08.06.2022
(21) Application number: 16196320.2
(22) Date of filing: 28.10.2016
(51) Int. Cl.: A61L 15/32, A61L 15/42, A61L 15/46, A61L 15/60

(54) **MULTI-LAYER WOUND CARE PRODUCT WITH PERFORATED COLLAGEN LAYER**
MEHRSCHICHTIGES WUNDPFLEGEPRODUKT MIT PERFORIERTER KOLLAGENSCHICHT
PRODUIT DE PARAGE MULTICOUCHE AVEC COUCHE DE COLLAGÈNE PERFORÉE

(43) Date of publication of application: 02.05.2018
(73) Proprietor: BSN medical GmbH, 22761 Hamburg (DE)
(72) Inventor: HEMMRICH, Karsten, 40667 Meerbusch (DE); BLUMENTHAL, Nils, 22765 Hamburg (DE)
(74) Representative: Cohausz & Florack

(56) References cited:
- WO-A1-2005/123170
- WO-A1-2006/062470
- US-A1- 2014 163 447
- US-A1- 2014 276 493
- US-A1- 2015 080 815

### SEQUENCE LISTING

<110> BSN Medical GmbH
<120> Multy-layer wound care product with perforated collagen layer
<130> 15213-PT-EP
<160> 46
<170> BiSSAP 1.3.5
<210> 1
   <211> 240
   <212> PRT
   <213> Streptococcus pyogenes
<223> "Triple helical protein"
<220>
   <223> Triple helical protein
<400> 1
   Gly Ser Pro Gly Leu Pro Gly Pro Arg Gly Glu Gln Gly Pro Thr Gly
   1               5                   10                  15
   Pro Thr Gly Pro Ala Gly Pro Arg Gly Leu Gln Gly Leu Gln Gly Leu
               20                  25                  30
   Gln Gly Glu Arg Gly Glu Gln Gly Pro Thr Gly Pro Ala Gly Pro Arg
           35                  40                  45
   Gly Leu Gln Gly Glu Arg Gly Glu Gln Gly Pro Thr Gly Leu Ala Gly
       50                  55                  60
   Lys Ala Gly Glu Ala Gly Ala Lys Gly Glu Thr Gly Pro Ala Gly Pro
   65                  70                  75                  80
   Gln Gly Pro Arg Gly Glu Gln Gly Pro Gln Gly Leu Pro Gly Lys Asp
                   85                  90                  95
   Gly Glu Ala Gly Ala Gln Gly Pro Ala Gly Pro Met Gly Pro Ala Gly
               100                 105                 110
   Glu Arg Gly Glu Lys Gly Glu Pro Gly Thr Gln Gly Ala Lys Gly Asp
           115                 120                 125
   Arg Gly Glu Thr Gly Pro Val Gly Pro Arg Gly Glu Arg Gly Glu Ala
       130                 135                 140
   Gly Pro Ala Gly Lys Asp Gly Glu Arg Gly Pro Val Gly Pro Ala Gly
   145                 150                 155                 160
   Lys Asp Gly Gln Asn Gly Gln Asp Gly Leu Pro Gly Lys Asp Gly Lys
                   165                 170                 175
   Asp Gly Gln Asn Gly Lys Asp Gly Leu Pro Gly Lys Asp Gly Lys Asp
               180                 185                 190
   Gly Gln Asn Gly Lys Asp Gly Leu Pro Gly Lys Asp Gly Lys Asp Gly
           195                 200                 205
   Gln Asp Gly Lys Asp Gly Leu Pro Gly Lys Asp Gly Lys Asp Gly Leu
       210                 215                 220
   Pro Gly Lys Asp Gly Lys Asp Gly Gln Pro Gly Lys Pro Gly Lys Tyr
   225                 230                 235                 240
<210> 2
   <211> 189
   <212> **PRT**
   <213> Clostridium perfringens
<223> "Triple helical protein"
<220>
   <223> Triple helical protein
<400> 2
   Gly Pro Arg Gly Pro Arg Gly Pro Gln Gly Glu Gln Gly Pro Gln Gly
   1               5                   10                  15
   Glu Arg Gly Phe Thr Gly Pro Gln Gly Pro Val Gly Pro Gln Gly Glu
               20                  25                  30
   Gln Gly Pro Gln Gly Glu Arg Gly Phe Thr Gly Pro Gln Gly Pro Ile
           35                  40                  45
   Gly Leu Gln Gly Glu Gln Gly Pro Gln Gly Glu Arg Gly Phe Thr Gly
       50                  55                  60
   Pro Gln Gly Pro Val Gly Pro Gln Gly Glu Gln Gly Pro Gln Gly Glu
   65                  70                  75                  80
   Arg Gly Phe Thr Gly Pro Gln Gly Pro Val Gly Pro Gln Gly Glu Gln
                   85                  90                  95
   Gly Pro Gln Gly Glu Arg Gly Phe Thr Gly Pro Gln Gly Pro Ile Gly
               100                 105                 110
   Pro Gln Gly Glu Gln Gly Pro Gln Gly Glu Arg Gly Phe Thr Gly Pro
           115                 120                 125
   Gln Gly Pro Ile Gly Pro Gln Gly Asn Gln Gly Pro Ile Gly Pro Gln
       130                 135                 140
   Gly Glu Gln Gly Pro Gln Gly Ala Thr Gly Pro Gln Gly Pro Gln Gly
   145                 150                 155                 160
   Pro Val Gly Pro Gln Gly Asn Gln Gly Pro Ile Gly Pro Gln Gly Pro
                   165                 170                 175
   Val Gly Pro Gln Gly Pro Gln Gly Gln Pro Gly Val Asn
               180                 185
<210> 3
   <211> 129
   <212> **PRT**
   <213> Methylobacterium sp. **4-46**
<223> "Triple helical protein"
<220>
   <223> Triple helical protein
<400> 3
   Gly Leu Pro Gly Pro Lys Gly Asp Pro Gly Pro Gln Gly Pro Ala Gly
   1               5                   10                  15
   Pro Lys Gly Glu Pro Gly Pro Lys Gly Glu Pro Gly Pro Lys Gly Glu
               20                  25                  30
   Pro Gly Pro Lys Gly Glu Pro Gly Pro Lys Gly Glu Pro Gly Pro Lys
           35                  40                  45
   Gly Glu Pro Gly Pro Lys Gly Glu Pro Gly Pro Lys Gly Glu Pro Gly
       50                  55                  60
   Pro Arg Gly Glu Ala Gly Pro Gln Gly Ala Leu Gly Pro Lys Gly Glu
   65                  70                  75                  80
   Ala Gly Ser Arg Gly Glu Pro Gly Pro Arg Gly Glu Pro Gly Pro Lys
                   85                  90                  95
   Gly Glu Ala Gly Leu Ala Gly Ala Pro Gly Pro Lys Gly Glu Ala Gly
               100                 105                 110
   Pro Arg Gly Pro Gln Gly Glu Arg Gly Pro Pro Gly Ala Pro Gly Ala
           115                 120                 125
   Ala
<210> 4
   <211> 246
   <212> **PRT**
   <213> Candidatus Solibacter usitatus Ellin6076
<223> "Triple helical protein"
<220>
   <223> Triple helical protein
<400> 4
   Gly Pro Ala Gly Pro Ala Gly Pro Gln Gly Pro Ala Gly Pro Ala Gly
   1               5                   10                  15
   Ala Gln Gly Pro Ala Gly Pro Ala Gly Pro Gln Gly Pro Ala Gly Pro
               20                  25                  30
   Gln Gly Ser Ala Gly Ala Gln Gly Pro Lys Gly Asp Thr Gly Ala Ala
           35                  40                  45
   Gly Pro Ala Gly Glu Ala Gly Pro Lys Gly Glu Thr Gly Ala Ala Gly
       50                  55                  60
   Pro Lys Gly Asp Thr Gly Ala Ala Gly Pro Ala Gly Pro Lys Gly Asp
   65                  70                  75                  80
   Thr Gly Ala Ala Gly Pro Ala Gly Pro Lys Gly Asp Thr Gly Ala Ala
                   85                  90                  95
   Gly Ala Thr Gly Pro Lys Gly Glu Lys Gly Glu Thr Gly Ala Ala Gly
               100                 105                 110
   Pro Lys Gly Asp Lys Gly Glu Thr Gly Ala Ala Gly Pro Lys Gly Asp
           115                 120                 125
   Lys Gly Glu Thr Gly Ala Ala Gly Pro Lys Gly Glu Lys Gly Glu Thr
       130                 135                 140
   Gly Ala Val Gly Pro Lys Gly Asp Lys Gly Glu Thr Gly Ala Ala Gly
   145                 150                 155                 160
   Pro Lys Gly Asp Arg Gly Glu Thr Gly Ala Val Gly Pro Lys Gly Asp
                   165                 170                 175
   Lys Gly Glu Thr Gly Ala Val Gly Pro Lys Gly Asp Lys Gly Glu Thr
               180                 185                 190
   Gly Ala Ile Gly Pro Lys Gly Asp Lys Gly Asp Lys Gly Asp Lys Gly
           195                 200                 205
   Asp Ala Gly Val Ala Gly Pro Gln Gly Ile Gln Gly Val Lys Gly Asp
       210                 215                 220
   Thr Gly Leu Gln Gly Pro Lys Gly Asp Ala Gly Pro Gln Gly Ala Pro
   225                 230                 235                 240
   Gly Thr Pro Gly Gly Gly
                   245
<210> 5
   <211> 117
   <212> PRT
   <213> Rhodopseudomonas palustris TIE-1
<223> "Triple helical protein"
<220>
   <223> Triple helical protein
<400> 5
   Gly Arg Pro Gly Pro Gln Gly Pro Arg Gly Arg Pro Gly Glu Pro Gly
   1               5                   10                  15
   Arg Pro Gly Pro Gln Gly His Pro Gly Arg Pro Gly Pro Glu Gly Pro
               20                  25                  30
   Arg Gly Lys Gln Gly Pro Val Gly Lys Pro Gly Pro Gln Gly Lys Ala
           35                  40                  45
   Gly Pro Gln Gly Lys Pro Gly Ile Ala Gly Lys Pro Gly Pro Asp Gly
       50                  55                  60
   Lys Pro Gly Pro Ile Gly Pro Gln Gly Lys Ala Gly Pro Gln Gly Pro
   65                  70                  75                  80
   Arg Gly Glu Gln Gly Leu Arg Gly Glu Gln Gly Pro Arg Gly Glu Gln
                   85                  90                  95
   Gly Pro Gln Gly Pro Arg Gly Glu Gln Gly Pro Arg Gly Glu Pro Gly
               100                 105                 110
   Pro Ala Gly Ala Leu
           115
<210> 6
   <211> 67
   <212> PRT
   <213> Streptococcus pyogenes
<223> "V domain of Scl 2"
<220>
   <223> V domain of Scl 2
<400> 6
   Glu Glu Asn Glu Lys Val Arg Glu Gln Glu Lys Leu Ile Gln Gln Leu
   1               5                   10                  15
   Ser Glu Lys Leu Val Glu Ile Asn Asp Leu Gln Thr Leu Asn Gly Asp
               20                  25                  30
   Lys Glu Ser Ile Gln Ser Leu Val Asp Tyr Leu Thr Arg Arg Gly Lys
           35                  40                  45
   Leu Glu Glu Glu Trp Met Glu Tyr Leu Asn Ser Gly Ile Gln Arg Lys
       50                  55                  60
   Leu Phe Val
   65
<210> 7
   <211> 86
   <212> PRT
   <213> Rhodopseudomonas palustris TIE-1
<223> "V domain"
<220>
   <223> V domain
<400> 7
   Pro Ser Ile Glu Gln Val Met Pro Trp Leu His Leu Ile Phe Asp Ala
   1 5 10 15
   Tyr Glu Asp Tyr Lys Ala Gln Arg Ala Arg Glu Ala Arg Glu Leu Glu
   20 25 30
   Glu Arg Leu Ala Ala Glu Ala Leu Glu Gln Ala Ala Arg Glu Ala Ala
   35 40 45
   Glu Arg Glu Val Ala Ala Ala Ile Glu Ala Ala Asn Ala Glu Ala Glu
   50 55 60
   Ile Met Leu Asp Asp Glu Thr His Ala Glu Gly Gly Lys Lys Lys Lys
   65 70 75 80
   Lys Arg Lys His Lys Asp
   85
<210> 8
   <211> 8
   <212> **PRT**
   <213> **Artificial** Sequence
<223> "Cleavage after **Gly4"**
<220>
   <223> Cleavage after **Gly4**
<400> 8
   Gly Pro Gln Gly Ile Ala Gly Gln
   1 5
<210> 9
   <211> 8
   <212> **PRT**
   <213> **Artificial** Sequence
<223> "Cleavage after **Gly4"**
<220>
   <223> Cleavage after **Gly4**
<400> 9
   Gly Pro Gln Gly Ile Trp Gly Gln
   1 5
<210> 10
   <211> 8
   <212> **PRT**
   <213> **Artificial** Sequence
<223> "Cleavage after **Gly4"**
<220>
   <223> Cleavage after **Gly4**
<400> 10
   Gly Asn Val Gly Leu Ala Gly Ala
   1 5
<210> 11
   <211> 6
   <212> **PRT**
   <213> Artificial Sequence
<223> **"Cleavage after Gly4"**
<220>
   <223> **Cleavage after Gly4**
<400> 11
   Gly Pro Gln Gly Ile Ala
   1 5
<210> 12
   <211> 6
   <212> **PRT**
   <213> Artificial Sequence
<223> **"Cleavage after Gly4"**
<220>
   <223> **Cleavage after Gly4**
<400> 12
   Gly Pro Gln Gly Ile Leu
   1 5
<210> 13
   <211> 6
   <212> PRT
   <213> Artificial Sequence
<223> "Cleveage after Gly4"
<220>
   <223> Cleveage after Gly4
<400> 13
   Gly Pro Gln Gly Leu Ala
   1 5
<210> 14
   <211> 6
   <212> **PRT**
   <213> Artificial Sequence
<223> **"Cleavage after Gly4"**
<220>
   <223> **Cleavage after Gly4**
<400> **14**
   Gly Pro Gln Gly Leu Leu
   1 5
<210> 15
   <211> 6
   <212> **PRT**
<213> Artificial Sequence
   <223> **"Cleavage after Gly4"**
<220>
   <223> **Cleavage after Gly4**
<400> 15
   Gly Pro Leu Gly Ile Ala
   1 5
<210> 16
   <211> 6
   <212> **PRT**
   <213> Artificial Sequence
<223> **"Cleavage after Gly4"**
<220>
   <223> **Cleavage after Gly4**
<400> 16
   Gly Pro Leu Gly Ile Leu
   1 5
<210> 17
   <211> 6
   <212> **PRT**
   <213> Artificial Sequence
<223> **"Cleavage after Gly4"**
<220>
   <223> **Cleavage after Gly4**
<400> 17
   Gly Pro Leu Gly Leu Ala
   1 5
<210> 18
   <211> 6
   <212> PRT
   <213> Artificial Sequence
<223> "Cleavage after Gly4"
<220>
   <223> Cleavage after Gly4
<400> 18
   Gly Pro Leu Gly Leu Leu
   1               5
<210> 19
   <211> 6
   <212> **PRT**
   <213> Artificial Sequence
<223> **"Cleavage after Gly4"**
<220>
   <223> **Cleavage after Gly4**
<400> 19
   Gly Pro Arg Gly Leu Gln
   1               5
<210> 20
   <211> 6
   <212> PRT
   <213> Artificial Sequence
<223> "Cleavage after Gly4"
<220>
   <223> Cleavage after Gly4
<400> 20
   Gly Pro Thr Gly Leu Ala
   1               5
<210> 21
   <211> 15
   <212> **PRT**
   <213> **Artificial** Sequence
<223> "MMP-cleavable peptide"
<220>
   <223> MMP-cleavable peptide
<400> 21
   Gly Pro Gln Gly Ile Ala Gly Gln Arg Gly Val Val Gly Leu Pro
   1               5                   10                  15
<210> 22
   <211> 15
   <212> PRT
   <213> Artificial Sequence
<223> "MMP-cleavable peptide"
<220>
   <223> MMP-cleavable peptide
<400> 22
   Gly Pro Gln Gly Leu Leu Gly Ala Pro Gly Ile Leu Gly Leu Pro
   1 5 10 15
<210> 23
   <211> 9
   <212> **PRT**
   <213> **Artificial** Sequence
<223> "MMP-cleavable peptide"
<220>
   <223> MMP-cleavable peptide
<400> 23
   Gly Glu Gln Gly Pro Gln Gly Leu Pro
   1 5
<210> 24
   <211> 12
   <212> **PRT**
   <213> **Artificial** Sequence
<223> "MMP-cleavable peptide"
<220>
   <223> MMP-cleavable peptide
<400> **24**
   Gly Pro Gln Gly Leu Ala Gly Gln Arg Gly Ile Val
   1 5 10
<210> 25
   <211> 35
   <212> PRT
   <213> Artificial Sequence
<223> "MMP-cleavable peptide"
<220>
   <223> MMP-cleavable peptide
<400> 25
   Gly Ala Gln Gly Pro Pro Gly Ala Pro Gly Pro Leu Gly Ile Ala Gly
   1 5 10 15
   Ile Thr Gly Ala Arg Gly Leu Ala Gly Pro Pro Gly Met Pro Gly Pro
   20 25 30
   Arg Gly Ser
   35
<210> 26
   <211> 12
   <212> PRT
   <213> Artificial Sequence
<223> "MMP-cleavable peptide"
<220>
   <223> MMP-cleavable peptide
<400> 26
   Gly Pro Leu Gly Ile Ala Gly Ile Thr Gly Ala Arg
   1 5 10
<210> 27
   <211> 24
   <212> PRT
   <213> Artificial Sequence
<223> "MMP-cleavable peptide"
<220>
   <223> MMP-cleavable peptide
<400> 27
   Gly Ala Gln Gly Pro Pro Gly Ala Pro Gly Pro Leu Gly Ile Ala Gly
   1 5 10 15
   Ile Thr Gly Ala Arg Gly Leu Ala
   20
<210> 28
   <211> 33
   <212> PRT
   <213> Artificial Sequence
<223> "MMP-cleavable peptide"
<220>
   <223> MMP-cleavable peptide
<400> 28
   Gly Pro Ser Gly Ala Glu Gly Pro Pro Gly Pro Gln Gly Leu Ala Gly
   1 5 10 15
   Gln Arg Gly Ile Val Gly Leu Pro Gly Gln Arg Gly Glu Arg Gly Phe
   20 25 30
   Pro
<210> 29
   <211> 12
   <212> PRT
   <213> Artificial Sequence
<223> "MMP-cleavable peptide"
<220>
   <223> MMP-cleavable peptide
<400> 29
   Gly Pro Gln Gly Leu Ala Gly Gln Arg Gly Ile Val
   1               5                   10
<210> 30
   <211> 24
   <212> **PRT**
   <213> **Artificial** Sequence
<223> "MMP-cleavable peptide"
<220>
   <223> MMP-cleavable peptide
<210> 31
   <211> 6
   <212> PRT
   <213> Artificial Sequence
<223> "Integrin binding site"
<220>
   <223> Integrin binding site
<400> 31
   Gly Phe Pro Gly Glu Arg
   1               5
<210> 32
   <211> 10
   <212> PRT
   <213> Artificial Sequence
<223> "Integrin binding site"
<220>
   <223> Integrin binding site
<400> 32
   Gly Glu Phe Tyr Phe Asp Leu Arg Leu Lys
   1 5 10
<210> 33
   <211> 5
   <212> PRT
   <213> Artificial Sequence
<223> "IKVAV motif"
<220>
   <223> IKVAV motif
<400> 33
   Ile Lys Val Ala Val
   1 5
<210> 34
   <211> 18
   <212> **PRT**
   <213> **Artificial** Sequence
<223> "Fibronectin binding domain"
<220>
   <223> Fibronectin binding domain
<400> 34
   Gly Leu Ala Gly Gln Arg Gly Ile Val Gly Leu Pro Gly Gln Arg Gly
   1 5 10 15
   Glu Arg
<210> 35
   <211> 12
   <212> PRT
   <213> Artificial Sequence
<223> "DDR2"
<220>
   <223> DDR2
<400> 35
   Gly Pro Arg Gly Gln Pro Gly Val Met Gly Phe Pro
   1 5 10
<210> 36
   <211> 6
   <212> PRT
   <213> Artificial Sequence
<223> "Collagen-like protein"
<220>
   <223> Collagen-like protein
<400> 36
   Gly Ala Ala Val Met Gly
   1 5
<210> 37
   <211> 8
   <212> PRT
   <213> Artificial Sequence
<223> "Collagen-like protein"
<220>
   <223> Collagen-like protein
<400> 37
   Gly Asp Ser Ala Val Ile Leu Gly
   1 5
<210> 38
   <211> 10
   <212> **PRT**
   <213> **Artificial** Sequence
<223> "Collagen-like protein"
<220>
   <223> Collagen-like protein
<400> 38
   Gly Asp Met Val Val Ser Arg Val Lys Gly
   1 5 10
<210> 39
   <211> 14
   <212> PRT
   <213> Artificial Sequence
<223> "Collagen-like protein"
<220>
   <223> Collagen-like protein
<400> 39
   Gly Arg Leu Val Asp Thr Gly Pro Gly Ala Arg Glu Lys Gly
   1 5 10
<210> 40
   <211> 43
   <212> **PRT**
   <213> **Artificial** Sequence
<223> "Collagen-like protein"
<220>
   <223> Collagen-like protein
<400> **40**
   Gly Ser Val Pro Asn Val Asp Arg Leu Leu Glu Thr Ala Gly Ile Lys
   1               5                   10                  15
   Ala Ser Ala Leu Arg Glu Ile Val Glu Thr Trp Asp Glu Ser Ser Gly
               20                  25                  30
   Ser Phe Leu Pro Val Pro Glu Arg Arg Arg Gly
           35                  40
<210> 41
   <211> 15
   <212> **PRT**
   <213> **Artificial** Sequence
<223> "Collagen-like protein"
<220>
   <223> Collagen-like protein
<400> **41**
   Gly Pro Gly Glu Phe Tyr Phe Asp Leu Arg Leu Lys Gly Pro Gly
   1               5                   10                  15
<210> 42
   <211> 16
   <212> **PRT**
   <213> **Artificial** Sequence
<223> "Collagen-like protein"
<220>
   <223> Collagen-like protein
<400> **42**
   Gly Gln Ile Ser Glu Gln Lys Arg Pro Ile Asp Val Glu Phe Gln Lys
   1               5                   10                  15
<210> 43
   <211> 6
   <212> **PRT**
   <213> **Artificial** Sequence
<223> "Ibeta-integrin binding domain"
<220>
   <223> Ibeta-integrin binding domain
<400> 43
   Gly Leu Pro Gly Glu Arg
   1 5
<210> **44**
   <211> 6
   <212> **PRT**
   <213> **Artificial** Sequence
   <223> "Ibeta-integrin binding domain"
<220>
   <223> Ibeta-integrin binding domain
<400> **44**
   Gly Phe Pro Gly Glu Arg
   1 5
<210> 45
   <211> 6
   <212> **PRT**
   <213> **Artificial** Sequence
<223> "Ibeta-integrin binding domain"
<220>
   <223> Ibeta-integrin binding domain
<400> **45**
   Gly Phe Pro Gly Glu Asn
   1 5
<210> 46
   <211> 16
   <212> PRT
   <213> Artificial Sequence
<223> "ECM retaining moiety"
<220>
   <223> ECM retaining moiety
<400> 46
   Asp His Leu Ser Asp Asn Tyr Thr Leu Asp His Asp Arg Ala Ile His
   1 5 10 15

## Description

### Field of the invention:

The invention relates to a multilayered wound care product comprising an upper liquid-absorbing layer, optionally an intermediate bacteria-adsorbing layer; and a liquid-permeable bottom layer comprising collagen, collagen-like protein or peptides comprising a matrix metalloprotease (MMP) cleavage site or a combination thereof. Also described herein is the use of said wound care product for plastic surgery, tissue engineering and for treatment of acute and chronic wounds and thereby also the use in the context of a vacuum therapy. The invention finally relates to a method of producing the wound care product.

### Background of the invention

The molecular and cellular mechanisms underlying the repair of the skin tissue has not been sufficiently investigated yet. As a consequence, the failure of the skin tissue to heal is still poorly understood, and current therapies are quite limited. Improper wound healing after trauma, acute illness, surgical interventions, or chronic disease conditions affects millions of people worldwide each year and is the consequence of misregulated elements of the tissue repair program that occurs in healthy tissue response. The repair response is a complex process which includes inflammation, angiogenesis, deposition of extracellular matrix and cell recruitment. Notably, the failure of one or more of the above stated cellular processes is generally linked to an underlying clinical condition, such as vascular disease, diabetes or aging, which are all frequently associated with healing pathologies (Eming et al., 2014; Science Translational Medicine, (6), 265: 1 - 15).

The process of wound healing is a dynamic one, and involves complex and phase-dependent interactions between cells, extracellular matrix (ECM), ECM components such as growth factors that work together to reconstitute tissue following injury (Clark et al., 2007, 127(5): 1018-1029). The wound healing processes can be classified into four phases, which are as follows: the inflammatory or exudative phase (cleansing phase), the granulation phase, the epithelization phase, and the reparative phase. The epithelization phase and the reparative phase are occasionally considered as one. The challenge of a successful wound care treatment is to specifically address the different requirements of the above described healing processes.

Within wound care management the wound infection, the protease imbalance and the exudate management proved to be three key aspects which have to be addressed for promoting the wound healing.

### Wound infection

Wound infection is one of the key problems in the management of the wound environment. An infection of the wound severely complicates the treatment and hinders the healing process by several aspects, such as reduction of the wound tensile strength, tissue damage, and furthermore induction of an unwanted inflammatory response. The enlarged bioburden of the wound leads to increased metabolic requirements of the tissue and stimulates a pro-inflammatory environment which in turn induces the recruitment of monocytes, macrophages and leucocytes, all of which have the potential to influence the healing in a negative way. As an example, excessive levels of the matrix metalloprotease MMP8 and neutrophil elastase in the wound tissue is a direct consequence of dominant neutrophil activity from either chronic inflammation or overeager antibacterial responses. These excessive protease levels lead to the degradation of growth factors and to the proteolysis of extracellular matrix proteins. Furthermore, the bacteria can induce the secretion of deleterious cytokines, which can induce vasoconstriction and thus reduced blood flow to the wound and thereby can cause systemic toxicity. At reduced levels, critical colonisation and localised, sub-clinical infection are also relevant factors in delayed healing, which ultimately increases healthcare costs and results in poor patient outcomes (Cutting 2011, Journal of Wound Care, JWC/BSN supplement; 3-19).

In sum, the control and especially the prevention of wound infection will optimise the potential for healing by maintaining an ideal wound environment and will manage associated health-related issues remain central to good wound care. This will ultimately result in significant cost savings in the health care system.

The use of antibiotics in wound care which are administered orally, intravenously and in some cases also topically has several disadvantages. At first the death and disruption of bacteria at the wound site results in the release of endotoxins and the deposition of cell debris, leading to subsequent inflammatory events locally and possibly systemically, even septic shock. Furthermore, when given systemically, antibiotics can affect the normal wound flora, leading to systemic complications and undesired side effects such as infections with *Clostridium difficile.* Antibiotics, when given systemically need an adequate blood supply to reach the infected target tissue and as a consequence may not be effective in treating wounds having a considerable necrotic burden or in treating patients with underlying arterial insufficiency. Finally, antibiotic resistance has become a severe problem in the field of wound care.

As an improvement the use of materials which absorb and retain (i.e. sequester) bacteria has been suggested. Hereby, the development of hydrophobically coated materials, e.g. by coating with dialkyl carbamoyl chloride (DACC) led to bacterial binding wound dressings such as Cutimed Sorbact (BSN medical GmbH, Hamburg, Germany).

### Protease management

The improper healing of chronic wounds has been linked to the disregulation of proteases and their respective protease inhibitors. In contrast to wound-healing process of acute wounds, whereby the tissue proteases are normally tightly regulated, it appears that for the chronic wound, the disruption of the synthesis and the subsequent activation of proteases is a key factor in wound pathogenesis. The so called "proteolytic imbalance" may be a result of the above described faulty regulation of inflammation and/or microbial contamination. It has been observed that matrix metalloproteinases, such as gelatinase A, gelatinase B and collagenase show increased levels in the exudate of chronic wounds as compared to exudates of acute wounds. Notably, chronic wounds show a prominent and continuing increase in serine proteases capable of degrading matrix components, such as fibronectin, as well as various key growth factors, all of which are required for cell growth and the remodelling of the extracellular matrix.

US 2015/080815 discloses a wound dressing comprising a wound contacting layer comprising a polysaccharide, preferably honey, and a MMP suppressing material, preferably collagen. This mixture of collagen and honey can be laminated on a substrate, e.g. a polyurethane foam or superabsorbent polymer film.

### Exudate management

The generation of wound exudate occurs as a consequence of vasodilation that occurs during the early inflammatory phase of the wound healing as induced by inflammatory transmitters such as bradykinin and histamine. The wound exudate is a serous fluid contained within the wound bed and represents a part of normal wound healing process in acute wounds. However, as soon as the wound becomes a chronic non-healing wound with abnormal, persistent inflammation or with an established infection established, exudate play a different role and will become a challenge in the wound care treatment. Notably, the wound exudate as generated in chronic wound contains components which are not observed in exudate from acute wounds such as proteolytic enzymes and other deleterious components. Hence, the chronic wound exudate has been regarded as 'a wounding agent in its own right' because it induces the degradation of growth factors and peri-wound skin tissue and makes the tissue vulnerable to further inflammation.

As a consequence of the above mentioned effects of wound exudate, an effective exudate management can has now become a key strategy within the treatment of chronic exuding wounds. First products show that the exudate management allows to accelerate the healing process, reduces the exudate-related problems such as infection and maceration, reduces frequency for changing the wound dressings and ultimately improves healthcare efficiency in view of costs and increased quality of life for the patient.

Wound dressings represent the dominant option for managing the wound exudate within the wound site. An optimal dressing should combine two different, in some respect contradictory effects: The dressings should remove excessive wound exudate but in the same instance should retain a certain wound moisture. Most of the dressing materials remove the wound exudate by absorption within the dressing or by allowing exudate evaporation. Many absorptive materials exhibit void spaces within their structure for taking up the fluids. Examples are viscose cotton, or polyester fabrics and polymer foams e.g. made from silicone or polyurethane or silicone. Notably these types of absorbers are not able to retain the wound exudate under pressure. In contrast there are improved dressing materials, such as hydrocolloids, alginates, carboxymethylcellulose (CMC) fibers and especially superabsorbent polymers (SAP) which have the absorb higher amounts of liquids and furthermore by forming a hydrogel can retain a high proportion of said absorbed exudate even under pressure. Notably, for strongly exuding wounds the combination of absorbing wounds dressing together with negative pressure has been observed to be beneficial.

### Management with topical negative pressure

NPWT has established itself as first choice of treatment for large wounds that cannot be closed without tension and/or that display significant challenges (contamination, high exudation). The NPWT is especially required in cases when frequent painful changes of the dressing are necessary or when leakage and soiling represent a clinical issue. The NPWT was found to be applicable to a broad spectrum of wounds including diabetic pressure and foot ulcers and has become the gold standard for treating sternal and open abdominal wounds.

Although there are wound care articles in the prior art which addresses some of the above discussed key aspects there is still no dressing that covers all of them.

Hence, there is still a need for an improved wound care product that addresses the different complex requirements for an improved wound healing. The objective of the present invention thus is to provide a wound care product which overcomes at least one of the above mentioned disadvantages.

This problem is solved by provision of a wound care product according to claim 1 and 2.

Specific embodiments are subject matter of further dependent claims.

### Summary of the invention:

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

In a first aspect the present invention provides a multilayered wound care product comprising:
(a) an upper liquid-absorbing layer;
(b) an intermediate bacteria-adsorbing layer; and
(c) a liquid-permeable bottom layer comprising collagen, a recombinant collagen-like protein of bacterial origin which comprises at least one MMP cleavage site or peptides comprising a matrix metalloprotease (MMP) cleavage site or a combination thereof; and optionally
(d) a cover layer situated above the upper liquid-absorbing layer;
wherein the liquid-permeable bottom layer is a continuous sheet-like layer having perforations or incisions and wherein the liquid permeability of said bottom layer is given by said perforations or incisions enabling the passage of liquid through said layer,
and wherein the collagen-like protein comprises the formula III:

(III) [(Gly-Xaa-Yaa)ₘ-(insert)ₙ]ₚ

where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site and wherein the overall content of Xaa and Yaa provides a proline rich structure where the total percentage of proline of all residues in the Xaa and Yaa positions is greater than 19%.

The wound care product of the present invention has several advantages over wound care products known in the prior art.

The multi-layer structure considers the complexity of wound pathophysiology and the different requirements during the wound-healing process. Hereby, the different layers address the following key problems:
The bottom collagen-like layer provides a valid protease management by providing a sacrificial proteolytic enzyme substrate for deleterious wound proteases such as MMP-2 and MMP-9. Thereby these proteases are removed from the wound site. As a consequence proteolytic fragments are generated that can enter the wound and support the wound healing process.

The intermediate bacteria-binding layer will sequester and remove microorganisms from the wound without leading to the release of endotoxins and bacterial debris.

The upper liquid absorbing layer removes excessive exudate and provides a moist wound healing.

As the inventors found out, this specific layer structure with the layering sequence results in an improved wound healing.

At first the top liquid-absorbing layer promotes a liquid stream from the wound through the bottom and intermediate layer. Thereby the proteases and the bacteria are co-eluted with the exudate and, as the inventors found out, they will be enriched in their target layers, namely, the proteases in the collagen layer and the bacteria in the intermediate bacteria-adsorbing layer.

The collagen, or collagen-like proteins and peptides closely resembles native ECM insofar as they can act as a scaffold for MMPs to bind to and break down collagen in the product. As a result reduced levels of MMPs are released back into wound as ECM-mimicking structure breaks down, rebalancing protease and growth factor levels in the wound. Furthermore due to further signalling molecules or parts thereof it can induce epithelial cells, fibroblasts and vascular endothelial cells to migrate into the bottom layer scaffold in which they than can proliferate.

An effective bacteria-adsorbing layer normally requires a direct contact with the wound. In context with the liquid absorbing layer and the induced exudate flow said layer is also effective when positioned as an intermediate layer whereby the efficacy is further enhanced by incisions or perforations of the bottom layer so that the intermediate layer will at least partially come into contact with the wound site.

Notably, the liquid absorbing layer also functions as a security measure if bacterial or protease content of the exudate exceeds the binding capacities of the bottom and the intermediate layer. Furthermore the liquid absorbing layer can bind toxins or bacterial debris which are released due to the death of microorganisms in the middle layer.

Although there is a general exudate flow from the wound to the absorbing layer, it still allows the release of trophic factors from the wound care article and especially from the bottom layer to the wound site. The reduced exudate resorption in later time of application coincides with the increased generation of trophic proteolytic fragments which are then more easily released to the wound site.

Furthermore it was found out that this multilayer structure is also suitable for the use in negative pressure wound therapy, especially when using polymeric foam as liquid absorbing layer.

Notably, the multi-layer structure of the present invention can be provided with additional layers such as, e.g., a cover layer.

By an individual selection of the bottom, intermediate and top layer the wound care product can easily be adapted to the specific requirements of the underlying injury. Hence, it offers an enormous flexibility of application while addressing the different phases of wound healing.

Since the wound care product of the invention is based on known components, it can be easily produced in a cost efficient manner.

### Detailed description of the invention

The inventive wound care product may be used as such, as a dressing or in combination with a backing known in the art. For example, the wound care product according to the present invention may be temporarily adhered to a backing. This backing may be removed from the wound care product once applied to the wound or after cellular ingrowth has started.

In a second aspect the present invention provides a multilayered wound care product comprising:
(a) an upper liquid-absorbing layer comprising a polymeric foam, wherein the foam is a polyurethane foam;
(b) a liquid-permeable bottom layer comprising collagen, a recombinant collagen-like protein of bacterial origin which comprises at least one MMP cleavage site or peptides comprising a matrix metalloprotease (MMP) cleavage site or a combination thereof; and optionally
(d) a cover layer situated above the upper liquid-absorbing layer

wherein the liquid-permeable bottom layer is a continuous sheet-like layer having incisions and wherein the liquid permeability of said bottom layer is given by said incisions enabling the passage of liquid through said layer and wherein the collagen-like protein comprises the formula III:

   (III) [(Gly-Xaa-Yaa)ₘ-(insert)ₙ]ₚ

   where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site and wherein the overall content of Xaa and Yaa provides a proline rich structure where the total percentage of proline of all residues in the Xaa and Yaa positions is greater than 19%,
   and wherein the protein collagen is not included in the upper liquid absorbing layer, and wherein the different layers are connected by the use of an adhesive.

By having a polymeric foam as liquid absorbing element this wound care article is especially suited for a use in negative pressure wound therapy.

In one embodiment of the invention, the wound care article has an intermediate bacteria-adsorbing layer.

### Liquid-absorbing layer

The liquid absorbing layer of the invention absorbs the liquid released by the wound which is mainly the wound exudate. As the most proximal layer with regard to the other layers of the invention it triggers a flow of the wound exudate through the other layers with the result that the MMP can be bound in the collagen layer and the bacteria in the bacteria-adsorbing layer. By absorbing the deleterious wound exudate and meanwhile providing a moist wound climate this layer promotes the process of wound healing.

The liquid absorbing layer of the wound care article can be made from various medically safe materials, such as open-cell foam plastic, gel or textile.

In one embodiment of the invention the liquid-absorbing layer comprises, consists essentially of or consists of at least one absorbent material selected from the group consisting of polymer foams, sponges, hydrocolloids, hydrogels and hydrophilic polymers such as superabsorbent polymers.

Preferably, it consists of at least one layer of a hydroactive fiber or another textile material and/or comprises hydroactive polymers.

Hydroactive polymers can be selected from the list consisting of superabsorbent polymers, alginates, hydrogel nanoparticles and combinations thereof.

The term "hydroactive polymers" is to be understood hereinafter as referring to polymers capable of binding large amounts of liquid. Said polymers preferably comprise superabsorbent substances comprising polyacrylates, modified cellulose and/or alginates.

In one embodiment of the invention, the protein collagen is not included in the liquid absorbing layer.

In a preferred embodiment of the invention the upper liquid-absorbing layer further comprises at least one antimicrobial active compound, preferably selected from the group consisting of bacteriocin like inhibitory substance (BLIS), silver based compound, biguanide salt like polyhexamethylbiguanide (PHMB), chlorhexidine, phenol derivatives, such as thymol and eugenol, chlorophenol and chlordiphenyl ether.

Liquid uptake capacity and liquid retention refer to the ability of an absorbent material to take up liquids and bind/retain them, respectively. It is generally expressed in grams of a liquid (for example distilled water or 0.9% saline) per gram of absorbent material. Both parameters are a function of the properties of the hydroactive polymer, of its proportion in relation to the overall product and also of the chemical and physical makeup of the overall product.

Superabsorbent polymers (SAPs) are manufactured polymers capable of imbibing liquids to a multiple-up to 1000 times-of their own weight. Chemically, they comprise a copolymer of acrylic acid (propenoic acid, C₃H₄O₂) and sodium acrylate (sodium salt of acrylic acid, NaC₃H₃O₂), wherein the ratio between the two monomers may vary. A so-called core-crosslinker (CXL) is additionally included in the monomer solution to join the resultant long-chain polymeric molecules together in places by means of a network of chemical bridges (known as "crosslinks"). These bridges render the polymer insoluble in water. On ingress of water or aqueous solutions of salt, the polymer bead swells up and causes this network of crosslinks to tauten at a molecular level, so the water is no longer able to escape unaided. The superabsorbent polymers may be present in the wound care article of the present invention in the form of a granular material, in the form of a powder, in the form of a loose aggregation, in the form of a compacted aggregation, in the form of a foam, in the form of fibers, in the form of a fibrous knit, laid or nonwoven web fabric and/or a fibrous wadding.

Alternatively, the chosen superabsorbents may be methylacrylic acid based, polyvinyl alcohol-maleic anhydride copolymers, polysaccharide-maleic anhydride copolymers, maleic acid derivatives, acrylamidopropanesulfonic acid copolymers, starch-acrylonitrile graft polymers, gelatinized starch derivatives, alkyl- or hydroxyalkylcellulose, carboxymethylcellulose, starch-acrylic acid graft polymers, vinyl acetate-acrylic ester copolymers, acrylonitrile copolymers or acrylamide copolymers.

Modified cellulose preferably comprises derivatives of cellulose, preferably sulfoalkylated cellulose and derivatives thereof, preferably cellulose ethyl sulfonates, carboxy alkylated cellulose, preferably carboxymethylcellulose, carboxyethylcellulose and/or carboxypropylcellulose, more complex cellulose derivatives, such as sulfoethylcarboxymethylcellulose, carboxymethylhydroxyethylcellulose, hydroxypropylmethylcellulose, and amidated cellulose derivatives, such as carboxymethylcellulose amide or carboxypropylcellulose amide. Carboxymethylcellulose takes the particular form of sodium carboxymethylcellulose and is commercially available under the name of "Hydrofaser". In hygiene and wound products, the fibers are converted into a planar matrix. As they take up liquid from the wound exudate, the fibers are gradually transformed into a gel pad wherein the liquid is held and not reemitted. The construction of the fibers in question is such that the wound exudate is only taken up in the vertical direction. As a result, the exudate will not flow over the wound edge as long as there is sufficient capacity. This is an effective way to prevent wound edge maceration.

Said hydroactive polymers may also comprise alginates. Alginates are obtained from brown algae and processed into a fibrous nonwoven web. Chemically, alginates are polysaccharides, specifically calcium and/or sodium salts of alginic acids. Alginates are capable of absorbing up to 20 times their own weight of liquid, the wound exudate being imported into the void spaces. The Ca²⁺ ions in the alginate lattice are exchanged for the Na⁺ ions from the exudate until the alginate has reached its point of saturation with sodium ions. In the process, the wound dressing swells up and the alginate fiber is transformed into a gel body as a result of the fibers swelling up.

Said hydroactive polymers may similarly also comprise hydrogel nanoparticles comprising hydroxy-terminated methacrylate monomers, such as 2-hydroxyethyl methacrylate (HEMA) and/or 2-hydroxypropyl methacrylate (HPMA), which are marketed as Altrazeal, for example.

It is particularly preferable for at least one wound care article of the set to further comprise a fibrous nonwoven web, comprising cellulose fibers, which hereinafter is also referred to as an absorbent body.

The absorbent body may preferably comprise an essentially flat absorbent body which is made of an absorbent material and which consists of an imbibing fibrous nonwoven web incorporating superabsorbent polymers dispersed therein. These may be present in the form of a granular material, in the form of a powder, in the form of a loose aggregation, in the form of a compacted aggregation, in the form of a foam, in the form of fibers, in the form of a fibrous knit, laid or nonwoven web fabric and/or fibrous wadding.

The absorbent body in question comprises at least one material selected from the group containing a mat, in particular fibrous nonwoven web airlaid from said yams or fibers of superabsorbent polymers having incorporated superabsorbent polymers, and/or a loose filling of superabsorbent polymers. Said airlaid mat may preferably include an essentially flat portion of absorbent material, said portion of absorbent material consisting for example of an imbibing fibrous nonwoven web formed from the fibers mentioned and having superabsorbent polymers dispersed therein.

This absorbent body may correspond to the absorbent insert that is present in an assignee wound dressing as for example disclosed in WO03094813, WO2007051599 and WO0152780 and as marketed under the trade name "Sorbion sachet".

The absorbent body in some other configuration may similarly form a core which comprises-optionally flock like-fibers or yams of superabsorbent polymers and also superabsorbent polymers in granule form, in which case the granules are adhered and/or fused to the fibers/yams at two or more heights, and the granules are distributed across more than 50% of the entire design height of a portion of the core at least, in which case there are mingled regions of granules and fibers. The weight fraction of superabsorbent polymers here may preferably be in the range between 10-25 wt. %. Similar designs are known from conventional incontinence materials and like sanitary napkins are known for their cushioning properties. A cover may be disposed around said core in an overlapping arrangement in regions, and which for example conceals an adhered seam and/or is part thereof.

It is particularly preferable for the absorbent body to comprise a fibrous web, preferably a nonwoven or airlaid web which consists of superabsorbent fibers ("SAPs", preferably polyacrylates) or contains same as a constituent part. The fibers may for example be blended with fluff pulp (cellulose) or with polyester fibers. A layered construction may be provided as an alternative or in addition.

The absorbent body in some other configuration may similarly contain at least one flat ply comprising superabsorbent-polymer fibers or yams having superabsorbent polymers adhered in granule form. This, in a preferred configuration, results in a construction for the body where there are at least two layers in that at least one top layer puts a layer comprising superabsorbent polymers underneath. A second, flanking top layer may optionally be provided.

In a preferred embodiment the fibers and superabsorbent polymers are merely fixed in the material by the adjacent position between the two materials. The optionally provided plurality of plies may in one preferred configuration also be physically compacted together by rolling, pressing, calendering or similar processes.

It is particularly preferable for said absorbent layer to have an area dimension of 5x10, 5x20, 10x20, 10x10, 10x15 or 15x15 cm.

The basis weight in this case may be in the range between ≥ 50 and ≤ 2000 g/m². Preference is here given to basis weights of 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, and/or 2000 each ± 25 g/m².

Thickness may here be in the range between ≥ 2 and ≤ 50 mm. Preference is given to thicknesses of 2, 3, 4, 5, 6, 7, 8, 9, 10, 12, 14, 16, 18, 20, 22, 24, 25, 26, 28, 30, 32, 34, 36, 38, 40, 42, 44, 46, 48, or 50 each ± 1 mm.

Liquid retention, measured with distilled water can be between ≥ 5 and ≤ 100 g/g. Preference in this case is given to values of 5, 6, 7, 8, 9, 10, 11, 12, 13, 14, 15, 16, 17, 18, 19, 20, 21, 22, 23, 24, 25, 26, 27, 28, 29, 30, 31,32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and/or 100 g/g.

The uptake capacity may here be in the range between ≥ 3 and ≤ 30 ml of 0.9% saline/m² at 0.2 psi pressure. Preference here is given to values of 3, 4, 5, 6, 7, 8, 9, 10, 11, 12, 13, 14, 15, 16, 17, 18, 19, 20, 21, 22, 23, 24, 25, 26, 27, 28, 29, and/or 30 ml of 0.9% saline/m². Alternatively, the uptake capacity can be in the range between ≥ 2 and ≤ 50 g of water/g. Preference in this case is given to values of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and/or 50 g of water/g.

The overall content of superabsorbent polymers may here be in the range between ≥ 5 and ≤ 100% w/w. Preference is here given to values of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70,71, 72, 73, 74, 75,76, 77, 78, 79, 80, 81, 82, 83, 84, 85, 86, 87, 88, 89, 90, 91, 92, 93, 94, 95, 96, 97, 98, 99 and/or 100% w/w.

Tensile strength may here be in the range between ≥ 5 and ≤ 80 N/5 cm. Preference is here given to values of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 and/or 80 N/5 cm.

Extensibility here can be in the range between ≥ 10 and ≤ 80%. Preference is here given to values of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 and/or 80%.

When superabsorbent fibers are used, the following types have turned out to be particularly advantageous in practice, as shown in the following two tables:

| **Type** | **1** | **2** | **3** |
|---|---|---|---|
| Structure 1 | Layered structure: thermo-bonded Airlaid with laminated nonwoven | 40% polyester short cut fiber, 60% SAF | Bicomponent fiber of a SAF and a thermoplastic material |
| Structure 2 | Bicomponent fiber of a SAF and a thermoplastic material + fluff pulp | Needle felt | Carded thermobonded nonwoven |
| SAF fiber type | 101/6/10 | 102/52/10 | 102/52/10 |
| Weight (g/cm²) | 560 | 540 | 1000 |
| Thickness (mm) | 6 | 5.4 | 20 |
| Absorption capacity | 31.2 I water/m² | >20 g water/g | >16 g water/g or 16.000 g water/m² |
| Absorption capacity under pressure (0.9% NaCl solution/m² at a pressure of 0.2 psi) | 16 | | |
| Total content of SAP (% w/w) | 18 | 40 | 50 |
| | | | |
| | | | |

| **Type** | **4** | **5** | **6** |
|---|---|---|---|
| Structure 1 | Layered structure: thermos-bonded Airlaid with laminated nonwoven | 25% polyester short cut fiber, 75% SAF | 40% polyester short cut fiber, 60% SAF |
| Structure 2 | Bicomponent fiber of a SAF and a thermoplastic material + fluff pulp | Needle felt | Needle felt101/6/10 |
| SAF fiber type | 101/6/10 | 102/52/10 | 102/52/10 |
| Weight (g/cm²) | 350 | 150 | 180 |
| Thickness (mm) | 3.5 | 2.4 | 3.8 |
| Absorption capacity | 19.5 I water/m² | >25 g 0.9% NaCl _{aq}/g | >17 g water/g or 6,400 g water/m² |
| Absorption capacity under pressure (0.9% NaCl solution/m² at a pressure of 0.2 psi) | 16 | | |
| Total content of SAP (% w/w) | 18 | 75 | 60 |
| Tensile strength (N/5cm) | | 16 ± 13 | 16 ± 13 |
| Elasticity (%) | | 60 ± 18 | 60 ± 18 |

Further preferred layers with superabsorbent polymers exhibit characteristics as shown in the following table:

| **Type** | **1** | **2** | **3** | **4** | **5** | **6** | **7** | **8** |
|---|---|---|---|---|---|---|---|---|
| Weight (g/m²) | 430 | 300 | 150 | 5 | 100 | 120 | 140 | 440 |
| Thickness (mm) | 1.3 | 1.2 | 0.9 | 0.7 | 0.7 | 0.76 | 1 | 1.2 |
| Liquid retention (g/g) | 28 | 33 | 28 | 15 | 25 | 28 | 11.5 | 38 |
| Tensile strength (N/5 cm) | 25 | 55 | 20 | 20 | 20 | 20 | 15 | 20 |
| Uptake capacity (g/g) | 45 | 200 | 50 | 20 | 40 | 50 | 28 | 55 |

### Foams

In an alternative embodiment the liquid absorbing layer comprises or consists of a foam, being preferably an open cell foam or even an reticulated foam. They create a moist environment and provide thermal insulation to the wound. A foam layer is especially suited for use in the reduced pressure treatment. Foams can form a soft cushion and easily absorb liquid which can thereafter be released by application of reduced pressure.

Foams are generally materials with cells (open, closed, or both) distributed over their whole mass. Materials thus generally have a raw density (in accordance with DIN 53420) which is lower than the density of the basic substance.

A cell is an individual cavity formed in the manufacture of the foam which is partially or fully enclosed by the cell walls and/or cell struts.

A closed cell is usually a cell which is completely enclosed by its walls and has no connection via the gas phase with the other cells. An open cell is generally a cell which is connected with other cells via the gas phase. In the context of this application, the term open-cell means that in the foam there is at least 60% open cells, preferably at least 90% open cells, even more preferably 98% open cells, in particular essentially 100% open cells with reference to the total number of cells.

The cell wall is generally taken to mean the wall enclosing the cell. The cell wall can also be referred to as the cell membrane. The cell strut is generally taken to mean the area of the cell wall which separates more than two cells. Cell struts are preferably at least 1.5 times the thickness, even more preferably at least twice the thickness of the rest of the cell wall.

A reticulated foam is taken to mean a foam which consists largely of cell struts.

Regarding the cross-linked polyorganosiloxane foam material the foam is preferably obtained by reaction by reaction of a curable mixture containing the components:
(i) a polyorganosiloxane containing one or more groups with a C₂-C₆ alkenyl group, preferably containing one or more vinyl groups,
(ii) a polyorganosiloxane containing one or more Si-H groups,
(iii) a foaming agent containing one or more OH groups, and
(iv) an organometallic catalyst.

In a preferred embodiment the above stated component (i) contains a polyorganosiloxane in accordance with general formula (I) 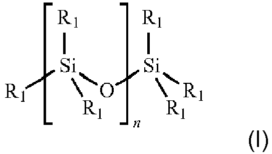 in which R₁ is independently C₁₋₆ alkyl, C₁₋₆ haloalkyl, aryl or C₂-C₆ alkenyl, provided that n has a value such that the viscosity of the polyorganosiloxane is in a range between 500 and 250,000 mPas and that the molecule contains at least one C₂-C₆ alkenyl group.

In a further preferred embodiment the component (ii) as stated above contains a polyorganosiloxane in accordance with the general formula (II): 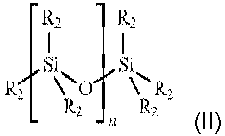 in which R₂ is independently C₁₋₆ alkyl, C₁₋₆ haloalkyl, aryl or hydrogen, provided that the molecule contains at least two hydrogen atoms bonded with silicon, whereby they are bonded to different silicon atoms.

For an advantageously effect within the wound care article the polymer foam of the invention has a specific tensile strength, a specific ductile yield and a specific hardness. In a preferred embodiment, the foam has a tensile strength of 100 kPa to 100 MPa, more preferably 500 kPa to 50 MPa, even more preferably 800 kPa to 10 MPa, measured in accordance with DIN 53571. Furthermore, the foam preferably has a ductile yield of 100% to 350%, more preferably from 160% to 320%, even more preferably from 180% to 300%, measured in accordance with DIN 53571. In addition to this, the foam preferably has a hardness of 20 to 70 Shore A, more preferably from 30 to 60 Shore A, even more preferably from 40 to 50 Shore A. measured in accordance with DIN 53505.

It is furthermore of advantage if the foam has a specific air permeability. In a preferred embodiment the foam has an air permeability of 1000 to 8000 l/(m²sec), more preferably from 2000 to 7500 l/(m²sec), even more preferably from 2500 to 7000 l/(m²sec), in particular from 3000 to 6500 l/(m²sec), measured in accordance with DIN EN ISO 9237.

It has also been demonstrated that the foam layer behaves advantageously if the foam has visco-elastic properties. This means that the behaviour of the foam under strain looks like a combination of an elastic solid and a viscous fluid. The visco-elastic behaviour can be characterized by a torsional vibration test in accordance with DIN 53445. It is preferred that the foam, when determined in accordance with DIN 53445 at 23 °C., has a mechanical loss factor of 0.1 to 1.0, more preferably from 0.15 to 0.8, even more preferably from 0.2 to 0.6.

In another embodiment of the invention the polymeric foam has a porosity with pore size ranging from about 10 µm to 500 µm, preferably from 20 µm to 120 µm.

It has also been demonstrated that the objects set out above can be solved unexpectedly advantageously if the foam has a raw density between 0.12 and 0.35 g/cm³, more preferably between 0.15 and 0.25 g/cm³, in particular between 0.18 and 0.22 g/cm³, measured in accordance with DIN EN ISO 845.

In a preferred embodiment the polymeric foam is used in a dry condition.

In a further preferred embodiment the polymeric foam layer has a thickness of 1 to 50 mm, preferably from 15 to 30 mm, and most preferably from 20 to 25 mm.

In another embodiment of the invention the polymeric foam is provided with further antioxidant, bactericidal or bacteriostatic agents as disclosed within the present application.

### Bacteria-adsorbing layer

According to at least the first aspect of the invention the wound care article comprises a bacteria-adsorbing layer to bind and subsequently remove microorganisms and hydrophobic endotoxins from wounds. Several microbial cell surface structures have been reported to express hydrophobic properties, and are therefore likely to mediate adhesion to substrates by hydrophobic interaction. Examples of hydrophobic tissue adhesions include fimbriae of Gram-negative bacteria, cell surface proteins of fungi, S-layer proteins (capsule-like polysaccharide surface coatings) and lipoteichoic acid of Gram-positive bacteria.

According to the invention the bacteria-adsorbing layer is also a liquid-permeable layer. This ensures that the liquid, namely the wound exudate, coming from the bottom collagen(-like) layer is further transported through the bacteria adsorbing layer to the top liquid absorbing layer.

The use of said bacteria-adsorbing layer has several advantages:
- Binding and removal of a broad range of detrimental microorganisms
- Binding and removal of bacterial toxins
- Leaves non-hydrophobic microorganisms in the wound to promote wound healing
- Low likelihood of spreading bacteria during a dressing change
- Non-allergenic
- Optimal binding capacity in a moist environment
- No development of antibiotic resistance.

In a preferred embodiment the bacteria -adsorbing layer is not only suitable for adsorbing bacteria but also other microorganisms such as viruses or fungi.

In order to adsorb bacteria said layer comprises or consists of a hydrophobic material. In the context of the present invention, the hydrophobic material is defined as a material with a contact angle (water) of more than 90°. In a preferred embodiment the hydrophobic material has a contact angle (water) of between 100° and 170°, more preferably of between 120° and 160° and most preferably of between 140° and 160°.

In one embodiment of the invention the intermediate bacteria-adsorbing layer comprises a hydrophobic or hydrophobized carrier material, which is preferably a hydrophilic carrier material treated with silicone, dialkyl carbamoyl chloride, alkyl ketene dimer (AKD), or other fatty acid derivatives such as stearylic acid.

In a preferred embodiment this hydrophobic material is generated by treatment of cellulose, cellulose acetate, cotton gauze or nonwoven with a compound containing hydrophobic groups.

In a preferred embodiment said hydrophobic compound is selected from the group consisting of the fatty ester dialkyl carbamoyl chloride (DACC), dioctadecyl-carbamoyl chloride, silicone and alkene ketene dimer (AKD)

In another preferred embodiment the intermediate bacteria-adsorbing layer is a cellulose layer, being preferably a biocellulose layer, coated with dialkyl carbamoyl chloride.

In a further preferred embodiment said layer consists of a acetate gauze or cotton gauze treated with DACC: In a preferred embodiment of the invention the intermediate bacteria-adsorbing layer further comprises at least one antimicrobial active compound, preferably selected from the group consisting of bacteriocin like inhibitory substance (BLIS), silver based compound, biguanide salt like polyhexamethylbiguanide (PHMB), chlorhexidine, phenol derivatives, such as thymol and eugenol, chlorophenol and chlordiphenyl ether.

### Further bioactive molecules

The layers of the wound care article of the invention might further contain bioactive molecules which might be already present in the respective layer(s) or alternatively added to the respective layer(s) after preparation. In a preferred embodiment these bioactive molecules are cytokines and/or chemokines. For example, the layer(s) may contain at least one compound from a group including fibroblast growth factor-2 (basic FGF), transforming growth factor-beta (TGF-beta) platelet derived growth factor (PDGF), cartilage derived growth factor (CDGF), epidermal growth factor (EGF), transforming growth factor alpha (TGF-alpha), thrombospondin, osteopontin, angiotensin converting enzyme (ACE), bone morphogenetic protein (BMP), NGF (nerve growth factor), tumor necrosis factor alpha (TNF-α), acidic fibroblast growth factor (aFGF), hepatocyte growth factor (HGF), insulin-like growth factors 1 and 2 (IGF-1 and IGF-2), stromal derived factor 1 alpha (SDF-1 alpha), ciliary neurotrophic factor (CNTF), neurotrophin-3, neurotrophin-4, neurotrophin-5, pleiotrophin protein (neurite growth-promoting factor 1), midkine protein (neurite growth-promoting factor 2), brain-derived neurotrophic factor (BDNF), tumor angiogenesis factor (TAF), corticotrophin releasing factor (CRF), interleukin-8 (IL-8), granulocyte-macrophage colony stimulating factor (GM-CSF), growth differentiation factor-9 (GDF9), myostatin (GDF-8), and/or vascular endothelial growth factor (VEGF). Further preferred is that the ECM material used in the present invention contains additional bioactive components including, for example, one or more of collagens, glycosaminoglycans, glycoproteins and proteoglycans. The ECM material may further include the basement membrane or parts thereof, which are typically made up mostly of type IV collagen, laminins and proteoglycans.

In various embodiments the bioactive molecules, being preferably growth factor(s) are released slowly and sustained over at least one day, or over at least 3 days or over at least 5 days, or over at least one week, or over at least two weeks, or over at least 3 weeks, or over at least one month, or aver at least two months, or aver at least three months, or over at least six months. Typically the growth factor(s) are released directly to the target tissue in the body.

### Antimicrobial compounds

Triacetin (glycerin triacetate) is an ester compound comprised of glycerin and acetic acid. It is antimicrobial, and is used as a softener, and due to its hygroscopic properties is also used as a moisturizing agent.

### Hemostatics

In a further embodiment the wound care product further comprises at least one hemostypic or haemostatic agent. These agents stimulate haemostasis, thereby supporting the healing process. Suitable examples for hemostatics are vitamin K, coagulating factors (e.g. factor VIII, factor IX), trans-4-aminomethyl-cyclohexan-carboxylic acid, epinephrine, adrenochrome, thrombin, fibrin, fibrinogen, cholesteryl sulfate, Extracts from pharmaceutical chamomile (Chamomilla recutita), leave extracts from dioecious nettle (Urtica dioica), carboxymethyl chitosan, polycarbophils (e.g., calcium carbophil), sephadex or debrisan.

### Further constituents

In addition, the wound care product may also contain analgesic substances, i.e. pain relievers. For this, essentially all of the substances that are listed in the main group 05 of the so-called "red list" may be considered. Particularly preferable thereby are specifically anti-inflammatory substances such as, so-called COX inhibitors or NSAID (non steroidal anti-inflammatory drugs), as well as, for example, propionic acid derivatives, such as naproxen, ibuprofen, ketoprofen, fenoprofen, flurbiprofen, dexibuprofen, or tiaprofenic acid, acetic acid derivatives such as diclofenac, alclofenac, etodolac, aceclofenac, sulindac, or indometacin, heterocyclic acetic acids such as ketorolac, arylalkanoic acids such as tolmetin, N-phenylacetic acids such as mefenatnic acid or flufenamic acid, salicylates such as acetylsalicylic acid (aspirin), salicylic acid, or diffunisal, pyrazolone derivatives such as phenylbutazone, oxicam derivatives such as piroxicam, tenoxicam, meloxicam, or lomoxicam, enolic acid derivatives such as aminopyrine or antipyrine, phenols such as acetaminophen and similar items. In addition, there are the COX-2-inhibitors such as rofecoxib, lumiracoxib or celecoxib.

Furthermore, substances which are not anti-inflammatory may also be used as pain relievers, such as, for example, opiates, local anaesthetics such as lidocaine, mepivacaine, prilocaine, procaine, syntocaine, tetracaine, gingicaine, articaine, bupivacaine, butanilicaine, chloroprocaine, or for example, polidocanol.

In addition, the wound care product may contain anti-inflammatory substances that, as the case may be, exhibit secondary analgesic properties, such as, for example, aside from the above mentioned, which in part are also anti-inflammatory analgesics, hormones, particularly cortisone and corticoids, specifically glucocorticoids (e.g. cortisone, cloprednol, prednisone, prednisolone, methylprednisolone, deflazacort, fluocortolone, triamcinolone, dexamethasone, betamethasone) and mineralocorticoids (e.g. aldosterone, desoxycorticosterone, fludrocortisone ).

As a rule, it may be beneficial to overdose the wound beyond the acute treatment needs with said substances or complexes of substances-particularly the nutrients, disinfectants and/or, as the case may be, the analgesics as, for example, portions of the substances will remain in the dressing. This overdosing serves, however, not to resolve the overall insufficiency of the nutrients in a patient, or to prevent systemic sepsis, because a systemic effect, as is mentioned above, is neither intended nor desired. Nevertheless, the selected dosage may be higher than the dosage, as will be explained in the following, which is the recommended oral or enteral daily dosage. This is useful in particular because, for example, in the end the application of the composition in a dressing should frequently remain on the dressing for a longer period of time.

If it is the case that compositions are used, for example, that contain a dietetic composition, it is particularly intended that the amount used, for example, per wound care product (e.g. foam pad, or dressing containing SAP) lies in the range between 10% and 200% of the DGE (Deutsche Gesellschaft für Ernährungsmedizin [German Society for Nutritional Medicine]) recommended daily dosage. It is particularly preferred that this lie in the range between 30% and 100% of the recommended daily dosage.

The said composition can, thereby, be incorporated in the dissolved form of dressing, for example, or, respectively, incorporated in the dressing in advance. Alternatively, the composition may be incorporated in the dressing in its dehydrated form.

In the case that, for example, compositions are used which contain disinfecting substances, it is ideally intended that the amount used, per dressing, for example, be increased by a factor of 10 over the recommended daily dosage per kg bodyweight.

In additional preferred versions, the wound care product of the invention contains in addition, one or more substance(s) which are selected from the group containing orthomolecular nutrients, nutraceuticals, phytochemicals, antioxidants, growth factors, petroleum based blistering compounds, methylxanthines, tallins, tacrolimus, pimecrolimus, ATP, urea, sympathomimetic drugs, parasympatholytics, activated carbon, octenidine, polyhexanide, homeopathic remedies, QIO, thickening agents, karaja, pectin, agar, aloe vera, haemostatics, animal saliva such as maggot or canine saliva, spider web proteins, collagen, hygroscopics, glycerin, biofilm harming substances, triacetin, zinc oxide, light absorbing components, odor inhibitors, gelling agents, exudation promoting substances, swelling reducing agents, radical scavengers, and/or honey or, respectively, its components. Most of these components belong to the definitions given above of nutrients, disinfectants, and/or proteases inhibiting substances or complexes of substances.

Orthomolecular nutrients in the framework of the concept of orthomolecular medicine, an alternative medical process largely influenced by Linus Pauling, are nutrients which are used, particularly vitamins and minerals, and may be administered in part in the intended high-dosage dosage regimen.

Nutraceuticals are nutrients which are enriched with additional substances (nutrition enriching agents), which should have a positive effect on health. These additives are primarily vitamins, minerals, bacteria cultures, and unsaturated fatty acids.

Phytochemicals are secondary plant material, in particular; these may be carotenoids, polyphenols and sterols. Among other properties attributed to said, are those of antioxidants and the fighting of free radicals, as well as the promotion of immunity and the reduction of cholesterol levels.

Antioxidants are substances which prevent the oxidation of sensitive molecules, particularly DNA and proteins. They usually function as radical scavengers. Antioxidants can be categorized as "antioxidants", "reducing substances" and "antioxidants with synergetic effects." A definition for the so-called true antioxidants is the mechanism whereby the chain reaction resulting from the scavenging of free radicals is blocked.

Examples of such antioxidants are BHA and BHT. In contrast to this, for example, ascorbic acid functions as a reducing agent by allowing lighter oxidation than that of the molecule being protected, thereby protecting said. Sodium EDTA belongs to this last group of synergistic antioxidants, for example, in that it enhances the antioxidant effect by bonding with metal ions.

In the framework of the present invention, the following antioxidants are to be taken into consideration: Antioxidants belonging to the vitamin E group, carotenoids, particularly lycopene and B-carotene, glutathione, transferrin, albumin, ceruloplasmin, hemopexin, haptoglobin, antioxidant enzymes, particularly superoxide dismutase (SOD), glutathione peroxidase (GPX), and catalase, tin chloride, ascorbic acid (vitamin C) and its derivative sodium L-ascorbate, calcium L-ascorbate, isoascorbic acid, sodium isoascorbate, and ascorbyl palmitate, butylated hydroxyanisole, butylated hydroxytoluene, calcium disodium EDTA, gallates, particularly propyl gallate, octyl gallate, and dodecyl gallate (lauryl gallate), lecithin, lactic acid, polyphosphates such as diphosphate, triphosphate, and polyphosphate, sulfur dioxide, sodium sulfite, sodium bisulfite, potassium sulfite, calcium sulfite, calcium bisulfite, potassium bisulfite, selenium, tocopherol(vitamin E), alpha-tocopherol, gamma-tocopherol, delta-tocopherol, tin II-chloride, citric acid as well as sodium citrate, calcium citrate, and reducing agents such as acetylcysteine.

Petroleum based blistering compounds have an exudation promoting effect as well as a necrolytic effect. For this reason, they may also function in particular as decontaminants.

One understands under this term the so-called black blistering ointments in particular, such as ichthyol based compounds, but also shale oil sulfonates. Shale oil sulfonates such as ammonium bituminosulfate, for example, are water soluble through a sulfonation process. The said compounds may be incorporated in accordance with the invention in a fatty or aqueous component of the covering of the dressing, and be applied as the first contact layer to a wound.

Methylxanthines are a group of alkaloids, which are usually used as mild stimulants as well as for treating bronchial asthma. They include caffeine, theophylline, and theobromine. Xanthines are purine derivatives. They have a constricting effect and tend to reduce swelling, so that, as the case may be, oedema in the affected area is reduced, and nutritional, disinfecting and/or proteases inhibiting substances are not diluted unnecessarily.

Tannins function as astringents, i.e. they serve to reduce oedema, are ant-inflammatory, antibacterial, antiviral, and neutralize toxins.

Tacrolimus (also FK506 or FK-506) is a macrolide from the bacteria *Streptomyces tsukubaensis.* Tacrolimus is used as, among other things, a selective immunosuppressive against rejection reactions in organ transplants. Tacrolimus is both immunosuppressive and antimicrobial. Its effects can be compared with those of the polypeptide cyclosporine, but may be used in smaller doses. Tacrolimus intervenes in the metabolic process of T-cells, and inhibits their activity. It bonds to the cytosolic receptor, a so-called immunophilin within the target cell. The complex comprised of immunophilin and tacrolimus adheres to the serine/threonine-protein phosphatase calcineurin. Calcineurin is thereby rendered inactive. The same basically applies for the substance tacrolimus.

ATP is a nucleotide, formed from the triphosphate of the nucleoside adenosine, and as such is an energy rich component of the nucleic acids DNA and RNA. ATP is however also the universal form of directly available energy in every cell and at the same time an important regulator of energy providing processes. ATP can be released from energy stores (glycogen, creatine phosphate) as it is needed. By adding ATP to the composition of the invention, an energy source free of glucose is made available, and is particularly useful in treatments where diabetes is present for improving the energy balance of the cells.

Urea has a high capacity for bonding with water and also exhibits keratolytic properties. In addition, it serves as a source of moisture for fighting atopic eczema and lichen diseases and is therefore particularly suited for use in a composition in accordance with the invention.

Necrolytes are agents which eat away at necrotic tissues. These may, for example, be the petroleum based blistering compounds described here. Other possible necrolytic agents are, for example, urea or animal saliva, both of which will be described below.

Sympathomimetics have a stimulating effect on the sympathetic portion of the autonomic nerve system. They affect an increase in blood pressure and pulse rate, a dilation of the bronchial passage, an overall improvement in performance and an increase in energy consumption. In combination with the composition of the invention, these substances reduce swelling as well as edema.

Parasympatholytics are medicines which counteract the action of the parasympathetic nervous system. The therapeutic use of parasympatholytics is complicated by insufficient organ selectivity. In this manner atropine, as a medicine for chronic obstructive bronchitis, promotes not only dilation of the bronchial tract, but also stimulates the heartbeat, dilation of the pupils, and a contraction of the smooth muscles. Use of these substances has a comparable effect to the sympathomimetics described above.

Activated carbon is a fine-grained carbon with a large internal surface, which is used in, among other things, chemistry, medicine, water and waste treatment as well as ventilation and air conditioning technology. When incorporated in a composition in accordance with the invention, it can contribute to bonding with toxins arising from metabolic processes and germs, and thereby, cleansing of the affected area.

Q10 or coenzyme Q10 is a quinone derivative with lipophilic terpenoid side chains, structurally related to vitamin and vitamin E. Coenzyme Q 10 is an essential electron and proton vector between the complex I or complex II and the complex III of the respiratory chains and can support the energy metabolism of the cells in the affected area through resorption with nutrients in the framework of a composition in accordance with the invention.

Thickening agents are added to solutions, which preferably are aqueous solutions, in order to increase their viscosity. They are mainly able to bond with water. Through extraction of unbonded water, the viscosity is increased. After a certain point has been reached, characteristic for each type of thickening agent, additional moisturizing effects occur which usually lead to an over proportional increase in viscosity. Thickening agents in combination with the composition of the invention allow for an adaptation to the surface of the wound, and a maximization of the resorption surface.

Suitable thickening agents are, for example, karaya (Indian tragacanth, karaya gum, E 416), a natural gum comprised of carbohydrates and galacturonic acids (secretion of the Indian sterculia tree), alginic acid, agar, carrageen, locust bean gum, guar gum, tragacanth, gum Arabic, Xanthan gum, karaya, tara gum, gellan, pectin, cellulose, cellulose ether, carboxymethyl cellulose, hydroxypropyl cellulose, hydroxypropyl methylcellulose, methyl cellulose, methyl ethyl cellulose, modified starch, egg yolk, roux, sago, and starch.

Pectins are vegetable polysaccharides, or specifically polyuronides, which for the most part are comprised of α-1.4-glycosidically linked D-galacturonic acid units. Many microorganisms are able to metabolize pectins. Due to their ability to create gels, pectins may also be used as a thickening agent in the manner indicated above. In addition, they are capable of functioning as chelating agents in the detoxification of heavy metal poisoning, and are therefore particularly suited for use in the framework of a composition in accordance with the invention.

Aloe vera is a plant from the aloe family which is produced in a gel of the same name, also called acemannan, having a long chain polysaccharide. This substance stimulates the immune system in in-vitro experiments, protects the cell membranes, and is antibacterial, antiviral, and antimycotic. This substance is absorbed into the body particularly well through the gastro-intestinal tract, and can also be used, however, in the affected area. In addition, aloe vera contains minerals (calcium, magnesium, zinc, selenium, and others), vitamins, amino acids, and secondary plant substances (flavonoids). The term "aloe vera" for the purposes of the present invention, may also refer to aloe vera extracts, the substances of which may be more easily absorbed by cells in the affected area.

The term "animal saliva" refers particularly to canine saliva and maggot saliva. Canine saliva, aside from having disinfecting agents, also contains necrolytic and granulation promoting substances, and in this manner, can accelerate the healing process. Maggot saliva is the saliva from insects used in the treatment of wounds, particularly maggots (notably, the common green bottle fly, *Lucilia sericata*). Said insects are able to cleanse chronic wounds of necrotic tissue and bacterial waste. Aside from the removal of wound debris and bacteria, the healing process and the regeneration of fresh tissue is stimulated and promoted by substances, particularly enzymes, which are contained in the saliva of the common green bottle fly.

With said saliva, it may be particularly intended that it be produced through recombinant processes. i.e. genetically.

Spider silk is comprised of long chain protein molecules. The threads produced in the salivary glands of spiders, due to the special molecular arrangement of the amino acids involved, are very flexible, extremely resilient, highly tensile, and at the same time highly elastic. The gossamer filaments are light and water tight, having, however, a high capacity for water absorption which is comparable to wool. They are resistant to microbial attacks and are therefore ideally suited for use in the composition of the invention, for example as sheathing material for a dressing containing the composition, or as a particulate, disinfecting substance. The term may also refer to synthetically, particularly recombinantly, produced spider silk.

Hygroscopy, in chemistry and physics, is the property of a substance (a hygroscopic) of bonding moisture in its vicinity (for the most part in the form of vapour in humidity). Hygroscopics can also, in particular, absorb exudate, and are therefore particularly suited for use in combination with the composition of the invention. Examples are super-absorbent polymers, glycerine, silicates such as silica gel, melissic acid and similar items. Said hygroscopics may also have, in particular, an exudation promoting effect, and thereby promote and enhance decontamination of the wound.

Furthermore, it may be intended that the composition of the invention also contain swelling reducing substances such as, for example, red eyebright *(Euphrasia officinalis)* extract, common sage *(Salvia officinalis)* or cowslip *(Primula veris*), vasoconstrictors such as oxymetazoline hydrochloride or xylometazoline hydrochloride or anti-oedemas. These may be helpful in reducing swelling in the affected area and to make oedematous fluid available, in order that they may be absorbed by an absorbent dressing.

Furthermore, it may be intended that the composition of the invention contain light absorbing components. These help to prevent the loss of structural integrity in light sensitive components (e.g. zinc oxide, vitamins).

Pigments, such as titanium dioxide for example, are particularly good components for light absorption. These can be incorporated in the sheathing or together with other components in one of the layers of the wound care product of the invention. In addition, they may be included in the packaging of the wound care product. Said may, however, be stored in the dark in general, in order to protect the contents contained therein from light.

Odour inhibiting substances absorb malodorous substances, restrain them, or prevent their existence, thereby improving the quality of life of the patients treated with the composition of the invention. These may be, for example, activated carbon, herbal extracts, perfumes and similar items.

Examples for odour inhibiting substances which can be used for the wound care article of the invention are cyclodextrin, activated carbon which can be present as powder, granulate, pellet, fiber, tissue etc., ricinol, ricinoleic acid and salts thereof, especially zinc salts, ricinol derivatives, zeolites, dispersions of organic nitrogen compounds, ion exchange materials as resins or granulates, diatomaceous earth, polymeric particles with high surface area, nitroimidazoles, preferably metronizadone.

In general, all of the disinfecting substances or complexes of substances have odour inhibiting properties.

Furthermore, the composition of the invention may contain gelling agents, such as, for example, agar, gelatines, acrylamides, agaroses, or UV-curable gelling agents. Using these, a gel may be formed on the wound, which on one hand acts as a cover for the wound, ensuring that the wound remains moist and protected, and on the other hand, acts as a carrier and donor of the substances or complexes of substances named, and ensures a supply of said to the wound.

Radical scavengers deactivate free radicals, which otherwise place biological tissue under oxidative stress, and initiate chain reactions which can generate damage to cells and tissues, particularly changes in the cellular DNA. These may be, in particular, epigallocatechin gallate, superoxide dismutase, glutathione peroxidase, vitamin A, vitamin C, vitamin E, coenzyme Q10 and anthocyanins. Bilirubin and uric acid are also able to neutralize free radicals, as well as the hormone melatonin. Radical scavengers are also frequently antioxidants. Particularly ideal thereby is notably the combination of vitamin C and vitamin E. A combination of this sort exhibits a particularly synergistic effect in regard to the antioxidative effect.

Honey consists 70-80% (mass) of inverted sugar, and contains, furthermore, enzymes, vitamins, amino acids, pollen, flavourings, and minerals. It has an antibacterial effect and is also hygroscopic, and for these reasons is particularly beneficial in combination with the wound care product of the invention.

Furthermore, it is particularly intended that there be one or more vitamins, selected from the group containing vitamin B12, vitamin D, vitamin C vitamin B1, vitamin B2, vitamin B6, niacin and/or folic acid in the vitamins.

An insufficiency of vitamin B12 (cobalamin) can lead to pernicious anemia (Perniziosa), a disease in the blood count and funicular myelosis. The causes of these insufficiencies may be an insufficient supply of nutrients, as has been observed with vegans, or insufficient resorption. With insufficient receptivity in the gastro-intestinal tract, the organism is lacking the intrinsic factor in its gastric juices, a glycoprotein which is produced by the parietal cells of the stomach and is essential for the metabolism of vitamin B12. The intrinsic factor binds cobalamin in a complex protected from the digestive system, and in this manner enables it to be transported in the stomach cells whereby vitamin B12 is able to arrive at the external tissues by bonding with other proteins (transcobalamin). A disturbance in the absorption in the terminal ileum may lead to insufficiency. Although a direct link to the healing process is unfamiliar to some sources, vitamin B12 is however one of the vitamins that typically need to be supplemented in older people.

Vitamin D is a collective name for a group of fat soluble vitamins which have numerous physiological effects. Its main representative in humans, vitamin D3 (or cholecalciferol) is a prohormone which the body produces in the skin with the aid of UV_{B} light or can be supplied nutritionally.

Vitamin C is an organic acid. Because it is easily oxidized, it has antioxidant properties. Its most important property is the physiological function as a vitamin. Insufficiency can result in scurvy in humans. Vitamin C is a radical scavenger and exhibits antioxidant properties (it functions, in other words as a reduction agent). It is an important co-factor in the hydroxylation reaction and, among other things, enables the body to produce its own collagen thereby. Furthermore, it plays an important role in the production of amino acids.

It protects other important metabolites and the genotype from oxidation through its antioxidant effects, or, respectively attacks from free radicals, which in the end means it provides protection to the cell from damage and thereby from cancer. Together with niacin and vitamin B6, vitamin C controls the production of L-carnitine, which is needed for the burning of fat in the musculature. In addition, it improves resorption of iron in the small intestine.

Thiamin or vitamin B1 is a water soluble vitamin in the B-complex having a weak, but characteristic odour and is particularly essential to the function of the nervous system.

Vitamin B 1 is necessary for the burning of carbohydrates, whereby it consumes itself as a co-enzyme. As the brain and the nerve cells are dependant on energy from carbohydrates, an insufficiency of thiamin particularly affects all brain and nerve functions.

VitaminB2 or riboflavin serves as a preliminary step for flavin co-enzymes (FAD, FMN), which play a particularly major role in oxidoreductases, for example in citric acid cycles. It assumes a central role thereby in metabolism. Riboflavin dissolves poorly in water, is sensitive to light, but is very resistant to heat. It contributes to a smooth complexion, and is involved in the regenerative mechanisms of the skin.

The phosphorylated vitamin B6 derivatives act as co-enzymes in approximately 100 enzymatic reactions. Nearly all reactions take place in amino acid metabolism. The pyridoxal phosphate (PLP or PALP) (a pyridoxine derivative) assumes another important function as a co-factor in the synthesis of δ-aminolevulinic acid, an intermediary product in the endogenous heme synthesis. Also noted is the participation of pyridoxal phosphate as a co-factor in the breakdown of animal starch (glycogens). Insufficiency results in the existence of dermatitides and growth disorders.

Niacin or nicotinic acid is a carboxylic acid of pyridine. Nicotinic acid is present in all living cells and is stored in the liver. It is an important building block of various coenzymes (NAD, NADP) and is of central importance in the metabolism of proteins, fats, and carbohydrates. It is less sensitive to heat, light, and oxygen than other vitamins in the B family. Nicotinic acid participates in the metabolism of proteins, fats, and carbohydrates. In the co-enzyme form NAD/NADP and their reduced forms NADH/NADPH, the so-called reduction equivalents, nicotinic acid is involved, for example, in the citric acid cycle and the respiratory chain. It is an antioxidant, and is involved in numerous enzymatic processes. Nicotinic acid is important for the regeneration of skin, muscle, nerves and DNA.

Folic acid is sensitive to light, oxygen, and heat, as well as being water soluble. An insufficiency of folic acid in the body affects the blood count in that it may lead to a hyperchromatic macrocytic anaemia.

Due to their metabolic-physiological characteristics, the vitamins named here, in particular either alone or in combinations, have a significant influence on the healing process, specifically because they improve the local nutritional situation, and thereby contribute to an improvement of the local cell metabolism.

Particularly preferred thereby is notably the combination of vitamin C and vitamin E. A combination of this sort has synergistic effects.

The substances named can be, without exception, as part of the wound care product of the invention incorporated in one or more of the layers.

### Liquid permeable bottom layer

According to the invention the liquid permeable bottom layer comprises, consists essentially of or consists of collagen, collagen-like proteins or peptides comprising a matrix metalloprotease (MMP) cleavage site or a combination thereof.

Since the bottom layer is liquid-permeable, the wound exudate can pass through said layer and reaches more proximal layer such as the intermediate bacteria-adsorbing layer and the top liquid-absorbing layer.

According to the claimed invention the liquid-permeable bottom layer is a continuous sheet-like layer having perforations or incisions which enable the passage of liquid through said layer.

In a preferred embodiment the liquid-permeable bottom layer comprises perforations. These apertures allow a direct contact of the intermediate layer with the wound site.

In another embodiment, the perforations or incisions are distributed in a regular manner over the area of the liquid-permeable bottom layer.

In a preferred embodiment the perforations each have an area of between 20 mm² and 400 mm² . Preference is here given to values of 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395 and/or, 400 mm².

In a preferred embodiment of the invention the liquid-permeable bottom layer has a thickness between 0.5 und 3 mm, preferably between 0.8 und 1.8 mm, und more preferably between 1.0 und 1.5 mm.

In a more preferred embodiment of the invention the liquid-permeable bottom layer is a grid-like structure. Hence, the sheet-like layer is equipped with perforations so that the sheet-like layer defines a grid or mesh with said perforations.

In more preferred embodiment the bottom layer is a grid-like structure with trigonal, tetragonal or hexagonal meshes.

In a further preferred embodiment the perforated bottom liquid-permeable layer allows a direct contact of the overlaying layer, being preferably the bacteria-adsorbing layer, with the wound site.

In one embodiment of the invention wherein the perforations account for between 20 to 80%, preferably between 30 to 70% and most preferably between 40 and 60% of the area of the liquid-permeable bottom layer.

The proportion of the area of the combined perforations as related to the total area of the bottom layer given in % can be in the range between ≥ 20 and ≤ 80%. Preference is here given to values of 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 and/or 80%.

The collagen as directly deriving from natural ECM comprises the relevant components for providing an ECM-like biological function. The further components, namely the collagen-like proteins or the MMP-cleavable peptides are modified to exhibit ECM-like properties.

### Collagen

In a preferred embodiment of the invention the hydrogel layer comprises collagen as ECM-mimicking structure.

In a preferred embodiment of the invention the collagen protein is selected from the group consisting of types I, II and III collagens and mixtures thereof.

Soluble collagens of the types I, II and III collagen are prepared by limited enzymatic digestion of tissue enriched in such types of collagen and subsequent formation of a collagen-based solution (i.e. a soluble collagen dissolved in a suitable solvent, such as diluted hydrochloric acid, diluted acetic acid or the like).

Insoluble collagens can be obtained from the following typical sources: type I collagen: bovine, chicken and fish skin, bovine and chicken tendons and bovine and chicken bones including fetal tissues; type II collagen: bovine articular cartilage, nasal septum, sternal cartilage; and type III collagen: bovine and human aorta and skin.

In another embodiment of the invention the collagen protein as used for the polymer layer is a human recombinant collagen.

The term "human recombinant collagen" refers to collagen manufactured by culturing a non-human organism genetically modified to express at least one human gene encoding a collagen. The human recombinant collagen is suitably selected from the group consisting of collagen type I, type II, type III, type IV, type V, type VI, type VII, type VIII, type IX, type X, type XI, type XII, type XIII, type XIV, type XV, type XVI, type XVII, type XVIII, type XIX, type XX, type XXI, type XXII, type XXIII, type XXIV, type XXV, type XXVI, and type XXVII. The collagen can be collagen of one type free of any other type, or can be a mixture of different types of collagen. Suitably, the collagen comprises or consists essentially of collagens selected from the group consisting of type I collagen, type III collagen and mixtures thereof.

Human recombinant collagen may be provided by any suitable method known in the art. For example, the step of providing human recombinant collagen may comprise the following protocol described in U. S. Pat. No. 5,962,648. Further recombinant processes are set forth in U.S. Pat. No. 5,593,859 and WO2004/078120.

Preferably, collagen is produced by culturing a cell which has been genetically modified to express at least one gene encoding the polypeptide comprising collagen and additional genes encoding the subunits of the post-translational modifying enzyme prolyl 4-hydroxylase, followed by the purification of the resultant collagen monomer therefrom. The recombinant collagen solution may be subsequently subjected to polymerization or cross-linking conditions to produce insoluble fibrous collagen.

Bovine collagen is a mixture of collagen type I (85%) and collagen type III (15%), a combination which is set by nature and cannot be varied. An advantage of recombinant collagen is that collagen type I and collagen type III are made independently of one another, and so any combination of type I and type III collagen can be made. The products according to the present invention may suitably comprise human collagen type I and human collagen type III in any ratio. For example, the products may comprise human collagen type I and human collagen type III in a ratio by weight of 100:0, 80:20, 60:40, 50:50, 40:60, 20:80 or 0:100, or anywhere in-between. Preferably, the ratio by weight of human collagen type I : human collagen type III is greater than about 50:50, and preferably it is greater than about 70:30, for example about 80:20. Suitably, the type I human recombinant collagen makes up at least about 75% by weight of the total human recombinant collagens in the material.

### Collagen-like protein

In one embodiment of the invention the ECM-mimicking structure is a collagen-like protein. In the context of the present invention the "collagen-like protein" is defined as a recombinant collagen-like protein of bacterial origin which comprises at least one MMP cleavage site. Typically, it represents a modular collagen-like protein that is stable at mammalian body temperature either in its native state or after chemical cross-linking and aggregates to form fibrils.

According to the claimed invention, the collagen-like protein comprises the formula III:

(III) [(Gly-Xaa-Yaa)ₘ-(insert)ₙ]ₚ

where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site.

The overall content of Xaa and Yaa provides a proline rich structure where the total percentage of proline of all residues in the Xaa and Yaa positions is greater than 19%, but optimally, though not exclusively, between 19.5 and 40%. Alternatively, or in conjunction with the proline concentration, the triple helical motif may also contain a concentration of charged residues (e.g. Asp, Glu, Lys, Arg, His) of greater than 14% and optimally, though not exclusively, between 14-35%. Such domains should also aggregate, either naturally or synthetically, at a neutral pH or otherwise using one or a variation of such protocols discussed herein or otherwise known in the art.

The triple helical domains may be isolated from one or multiple pathogenic or non-pathogenic bacterial organisms. By way of example, the triple helical domains can include domains derived from *Streptococcus pyogenes, Clostridium perfringens, Methylobacterium sp.4-46, Solibacter usitatus* Ellin6076 or *Rhodopseudomonas palustris* tie-1, which exhibit the desired heat stability in either its native state or after stabilization by chemical cross-linking. Such sequences may include those identified in U.S. Patent No. 6,953,839 or WO2010/091251. Alternatively, each triple helical domain may include repeats, fragments, homologues or combinations of the foregoing peptide sequences.

In a preferred embodiment of the invention the triple helical domains of the collagen-like protein are independently selected from the group consisting of SEQ ID No. 1 to 5.
CL triple helical protein from *Streptococcus pyogenes* (SEQ ID No. 1)
Triple helical protein from *Clostridium perfringens* (SEQ ID No. 2):
Triple helical protein from *Methylobacterium sp.* 4-46 (SEQ ID No. 3):
Triple helical protein from *Solibacter Usitatus Ellin6076* (SEQ ID No. 4):
Triple helical protein from *Rhodopseudomonas palustris tie-1* (SEQ ID No. 5):

Also important to the stabilizing structure of the collagen-like products of the instant invention is the proper formation of the triple helical structure. While some triple helical domains are able to be formed using the native mechanisms of the expression vehicle, proper folding of the recombinant bacterial collagen-like protein represented by formula III may also be assisted using globular or variable (V) domains. Such globular domains may be expressed directly or indirectly to the N-terminus and/or the C-terminus of the triple helical domain and can facilitate both post-translational folding and refolding following heat denaturation. One non-limiting example of an N-terminus domain is the entirety of or a portion of a variable (V) domain the Scl2 sequence found in *Streptococcus pyogenes,* which may be provided as the following peptide sequence:

A non-limiting example of a C-terminus variable domain or V domain may be isolated from the organism *Rhodopseudomonas palustris* tie-1, which may be provided as the following peptide sequence:

Further stabilizing structures such as "coiled coil forming sequences", C-propeptides or "foldons" may include those identified in WO2010/091251.

### Peptide

In one embodiment of the invention, the ECM-mimicking structure is a peptide comprising a MMP cleavage site.

In one preferred embodiment of the invention, the ratio of the ECM-mimicking peptide to the carrier material such as collagen or biodegradable fibrous material is between 0.5:1 and 1:1.

According to the invention, the term "peptide comprising a MMP cleavage site" is defined as an oligo- or polypeptide with less than 100 amino acids, preferably with less than 90, 80, 70, 60 or 50 amino acids, and more preferably with less than 40 or 30 amino acids, whereby the peptide is not a fragment of a collagen protein according to the invention and furthermore not a collagen-like protein according to the invention or fragment thereof. As used in the present invention this peptide is also denominated as "MMP-cleavable peptide"

In a preferred embodiment of the invention, the MMP cleavage site is given by one of the following amino acid sequences as given in single letter code:

| | | |
|---|---|---|
| • | GPQG↓IAGQ | (SEQ ID No. 8), |
| • | GPQG↓IWGQ | (SEQ ID No. 9), |
| • | GNV↓LAGA | (SEQ ID No. 10), |
| • | GPQG↓IA | (SEQ ID No. 11), |
| • | GPQG↓IL | (SEQ ID No. 12), |
| • | GPQG↓LA | (SEQ ID No. 13), |
| • | GPQG↓LL | (SEQ ID No. 14), |
| • | GPLG↓IA | (SEQ ID No. 15), |
| • | GPLG↓IL | (SEQ ID No. 16), |
| • | GPLG↓LA | (SEQ ID No. 17), |
| • | GPLG↓LL | (SEQ ID No. 18), |
| • | GPRG↓LQ | (SEQ ID No. 19), |
| • | GPTG↓LA | (SEQ ID No. 20), |
| • | GPQGIAGQRGWGLP | (SEQ ID No. 21) |
| • | GPQGLLGAPGILGLP | (SEQ ID No. 22) |
| • | GEQGPQGLP | (SEQ ID No. 23) |
| • | GPQGLAGQRGIV | (SEQ ID No. 24) |
| • | GAQGPPGAPGPLGIAGITGARGLAGPPGMPGPRGS | (SEQ ID No. 25) |
| • | GPLGIAGITGAR | (SEQ ID No. 26) |
| • | GAQGPPGAPGPLGIAGITGARGLA | (SEQ ID No. 27) |
| • | GPSGAEGPPGPQGLAGQRGIVGLPGQRGERGFP | (SEQ ID No. 28) |
| • | GPQGLAGQRGIV | (SEQ ID No. 29) |
| • | GPSGAEGPPGPQGLAGQRGIVGLP | (SEQ ID No. 30) |

In a preferred embodiment of the invention the MMP-cleavable peptide or the collagen like protein comprises one or more of the following:
a. a cellulose binding domain (CBD)
b. a heparin binding dipeptide motif (BA)ₙ, wherein B is an amino acid with a positively charged side chain, A is alanine an n is an integer between 4 and 20;
c. a pH-labile chemical linker which is preferably to the C-terminally or N-terminally attached to said peptide;
d. an integrin binding site, being preferably an RGD motif or having an amino acid sequence of GFPGER (SEQ ID No. 31) or GEFYFDLRLK (SEQ ID No. 32);
e. a photo-cleavable linker which is preferably C-terminally or N-terminally attached to said peptide;
f. an IKVAV (SEQ ID No. 33) motif;
g. a fibronectin binding domain, having preferably the amino acid sequence of GLAGQRGIVGLPGQRGER (SEQ ID No. 34);
h. a Discoidin domain-containing receptor 2 (DDR2) domain, having preferably an amino acid sequence of GPRGQPGVMGFP (SEQ ID No. 35);
i. a non-collagen-natural break having a peptide sequence spaced between two glycine residues of the insert sequence of the collagen-like protein; having an amino acid sequence of SEQ ID No. 36 to 42;
j. a heparin binding domain;
k. an α1β-integrin binding domain, being preferably GLPGER, GFPGER or GFPGEN (SEQ ID Nos. 43-45)
l. an ECM retaining moiety comprising the amino acid sequence DHLSDNYTLDHDRAIH (SEQ ID No. 46).

In a preferred embodiment of the invention the MMP-cleavable peptide or the collagen-like protein are covalently or non-covalently bound to a polymeric fiber material such as cellulose or nanocellulose, and hereby preferably by the use of a cellulose-binding domain (CBD).

CBDs adsorbs specifically and tightly on cellulose. Thus, they are a useful tool to address a bioactive peptide to the cellulose surface, in a specific and flexible way. By binding bifunctional CBD containing recombinant proteins, the (nano) cellulose can be provided with further functionalities such as binding domains for ECM proteins such as fibronectin or cell membrane proteins such as integrins.

Chimeric proteins with a CBD have been disclosed in several US. patents such as US. Pat Nos. 5,202,247 B1 or US 6,407,208 B1. Moreover, Andrade et al., Inc. J Biomed Mater Res 92A: 9-17, 2010, discloses a bifunctional protein with CBD fused to RGD which improves the affinity of fibroblasts for bacterial cellulose.

In a further embodiment of the invention, the MMP-cleavable peptide or the collagen-like protein further contains a repetitive dipeptide motif (BA)ₙ, wherein B is an amino acid with a positively charged side chain, A is alanine and n is a number from 4 to 20, indicating the number of individual repeating dipeptide modules (BA) within the dipeptide motif (BA)ₙ. The amino acid B is preferably arginine, with the one-letter code R, or lysine, indicated by the one-letter code K. Except for the L- and D-variants of arginine and lysine as natural amino acids, moreover generally all non-natural amino acids which are positively charged (basic) are suitable as amino acid B. The repetitive dipeptide motif represents a minimal binding motif for highly negative charged oligo- or polysaccharides such as heparin which enables the incorporation of heparin within the nanocellulose layer. Preferred repetitive dipeptide motif (BA)ₙ are disclosed in WO2014/040591.

According to another embodiment of the invention, the MMP-cleavable peptide or the collagen-like protein comprises a light-cleavable chemical linker, an enzymatically cleavable linker or a pH-sensitive chemical linker or a combination thereof. Due to this linkers the peptide or the collagen-like protein could be cleaved off the nanocellulose or alternatively certain functional domain could be cleaved from the nanocellulose binding peptide or the collagen-like protein in order to control the functionality of the peptide.

In another embodiment of the invention, the MMP-cleavable peptide or the collagen-like protein further comprises an oligonucleotide sequence as an enzymatically cleavable linker, which is a substrate for nucleases.

In an embodiment of the invention, the MMP-cleavable peptide or the collagen-like protein further comprises an integrin binding domain, having preferably the sequence cycloRGDyK.

The MMP-cleavable peptide or the collagen-like protein might contain insert sequences to improve the bendability or elasticity of the biomaterial or otherwise serve as a natural binding domain or biological cleavage sequence. Natural breaks or interruption sequences, for example, may include those of non-fibrillary human collagens, which are typically provided as 1 to 50 amino acids spaced between two glycine residues. While the instant invention is not so limited, examples of such sequences include those provided as follows (SEQ ID Nos. 31 to 37) as well as repeats, fragments, homologues or combinations thereof:

| | | |
|---|---|---|
| • | GAAVMG | (SEQ ID No. 36), |
| • | GDSAVILG | (SEQ ID No. 37), |
| • | GDMVVSRVKG | (SEQ ID No. 38), |
| • | GRLVDTGPGAREKG | (SEQ ID No. 39), |
| • | GSVPNVDRLLETAGIKASALREIVETWDESSGSFLPVPERRRG | |
| • | | (SEQ ID No. 40), |
| • | GPGEFYFDLRLKGPG | (SEQ ID No. 41), |
| • | GQISEQKRPIDVEFQK | (SEQ ID No. 42). |

In an alternative embodiment of the invention particulate ECM, collagen protein, collagen-like protein and MMP-cleavable peptide are not bound to the nanocellulose but are separated within the nanocellulose layer.

### Cover layer

In addition the wound care system and/or the wound care article can comprise an additional cover layer. Thus, in the overwhelming majority of cases it is considered desirable for a wound dressing to be available which always has a dry outer side (i.e. side facing away from the wound or the wound contact layer). The penetration of dirt and bacteria into the wound dressing from outside and in the worst case reaching the wound should also be prevented. This aim can be achieved by, for example, applying a fluid impermeable continuous protective film (hereinafter also referred to as backing film) as cover layer, whereby in a practical manner the backing film is water vapour-permeable. This layer which, as described above, should typically be impermeable to bacteria, adjoins the distal surface of liquid-absorbing layer. In an advantageous embodiment of the invention the film is only bound to the distal surface of the absorbing layer in a manner so that the film penetrate into the pores, cells or other intermediate spaces. The cover layer can be transparent to allow the level of filling or moisture in the wound dressing or the status of the wound to be assessed without having to remove the dressing. The cover layer can be filled with coloring agents. In general the film has a thickness of 10-500 µm and typically 15 to 45 µm, whereby film thicknesses of 30 ± micrometers are used in particular.

Films of this type are known from the prior art and comprise, for example, polyurethane-based films, such as a polyurethane film supplied by Exopack Advanced Coatings (Wrexham, UK) under the product name INSPIRE^{®}, or elastomer polyesters or mixtures of polyurethane with polyesters and/polyvinyl chloride and polyether amide block copolymers. Alternatively the backing layer can be a water-repellent and water vapour-permeable polyurethane foam with essentially closed cells, such supplied, for example, by Scapa (Greater Manchester, UK) under the product name Medifix.

For the purposes of the present application a polyurethane film is used as these films have good elastic properties and, in particular, exhibit form fitting properties as well as a high level of stretchability.

Suitable films have a moisture-vapour transmission rate (MVTR) of 500 to 14600 gm⁻²/24 hours, typically 1000 to 2700 gm⁻²/24 hours at 38 °C. Higher MVTR values can be advantageous in order to delay the saturation point of the wound dressing in strongly secreting wounds. Low MVTR values can be beneficial in assuring a moist microenvironment around the wound in the case of low-secretion wounds.

On the distal surface of the absorption layer the cover layer can be laminated in any known way. For example lamination can take place by means of heat or ultrasound or by means of an additional continuous and discontinuous adhesive layer arranged between the cover layer and the absorption layer.

Depending on the intended purpose of use it may be necessary to use film of a different thickness or to combine several layers/film. Thus, it may be advantageous to provide the above-described backing film with a carrier layer in order to guarantee a particular mechanical strength and thus prevent wrinkling of the backing film. In general the thickness of the entire layer, (i.e. the film and, if applicable, the carrier and additional layer(s) should be in a range of 5 to 2000 micrometers and typically in a range of 5 to 1000 micrometers. The layer and/or the outermost film should, for practical purposes, exhibit a low coefficient of friction and, for example, not catch on textiles or clothing, rub on them or negatively interact with textiles in general.

### Further characteristics of the wound care product

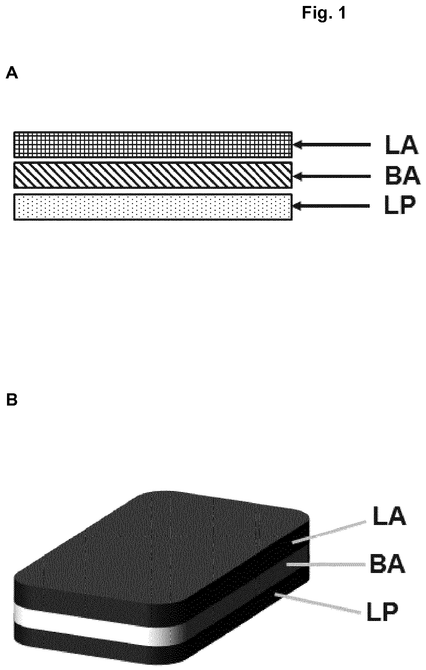

The dimension of the surface area of the wound care product may be selected according to the size of the wound. Typically, a wound care product of the present invention may have a surface area of from 5 cm² to 400cm², and particularly of from 25 cm² to 200cm², such as 100cm². Also the shape of the wound care product according to the present invention may vary with respect to the wound to be treated, and the present invention encompasses, for example, rectangular, square-, circle- or oval-shaped wound care products. For example, the wound care product may have a rectangular shape with rounded corners as depicted in Fig. 1B.

In a preferred embodiment of the invention the wound care product has a moisture vapor transmission rate (MVTR) as measured according DIN ISO 13726-2 of more than 1000 g/m²/24h, preferably of more than 1500 g/m²/24h, and preferably of more than 2000 g/m²/24h. The parameters are a target for the test MVTR upright. And MVTR inverted is >20.000 g/m²/24h. For a wet product or hydrogel the DIN ISO 13726-2 should be adapted to measure only the vapor transmission rate and not a combination of dry loss and vapour transmission rate.

### Bonding of the different layers

In a preferred embodiment of the invention the different layers of the multi-layered wound care article are connecting by use of an adhesive.

In preferred embodiment the adhesive connecting the bottom liquid-permeable layer with the overlying layer, which is preferably the intermediate bacteria-adsorbing layer, is a biodegradable adhesive. The biodegradable adhesive is selected from the group consisting of fibronectin; lectins; starch-based polymers such as thermoplastic starch, starch synthetic aliphatic polymer blends, starch-polybutylene succinate (PBS)/polybutylene succinate adipate (PBSA) polyester blends and starch-ethylene vinyl alcohol (EVOH) or polyvinyl alcohol (PVOH) blends; caseins; castor oil and soybean-based polyols, polylactic acids, material based on polysaccharides, animal proteins, plant proteins, and animal connective tissue.

In a more preferred embodiment jelly glue is used as biodegradable adhesive.

In another embodiment of the invention the adhesive connecting the top liquid -absorbing layer with the underlying layer is a non-biodegradable adhesive. This assures a solid and durable connection of the layers. As an example for such a non-biodegradable adhesive, silicone is mentioned.

In a further preferred embodiments the adhesive (irrespective of being a biodegradable or non-biodegradable adhesive) is not applied over the whole of the surface but rather over a restricted area over the surface such as in stripes, a mesh-like structure or in a punctuate structure. This ensures that the different layers maintain a certain horizontal flexibility and furthermore that the exudate can pass without hindrance trough the different layer.

In a preferred embodiment of the invention the an upper liquid-absorbing layer, the intermediate bacteria-adsorbing layer, and a liquid-permeable bottom layer are bonded at the border portion or at the edges of said layers, whereby the layers are adhered to the respective neighboring layer, to the upper cover layer or to a further outermost non-woven layer as wound contacting layer.

### Use of the wound-care product

The wound care product of the invention is particularly useful in the treatment of acute wounds, burn wounds, chronic wounds, and/or surgical wounds. The wound care product of the present invention may further be used in plastic surgery as well as for tissue engineering.

As such it can be used for the treatment of burns, partial and full-thickness wounds, pressure ulcers, venous ulcers, arterial ulcers, diabetic ulcers, chronic vascular ulcers, draining wounds, tunneled or undermined wounds, surgical wounds such as donor sites/grafts, post-Mohs surgery, post-laser surgery, podiatric dehiscence, and wound dehiscence, trauma wounds such as abrasions, lacerations, first, second, or third degree burns, and skin tears.

In the field of tissue engineering the wound care product of the invention can be used for remodelling of soft tissue, bone, cartilage, ligaments and tendons or dental applications.

For the medical use, it is required that the wound care product of the invention is provided in sterile form. This can be achieved by packaging the sterile product in a bacterial tight material with a marking on the packing that the product is sterilized. Bacterial tight materials are well known to the person skilled in art.

In observing the healing processes, four phases of the healing processes may be distinguished, specifically: the inflammatory or exudative phase (cleansing phase), the granulation phase, the epithelization phase, and the reparative phase. The epithelization phase and the reparative phase are occasionally considered as one.

### 1. Cleansing Phase

Particularly with acute wounds, hemostasis is of primary importance directly after the wound has occurred. The complement system (coagulation cascade) is activated, and loss of blood is limited by thrombocytes and aggregation of fibrin. This is supported by a simultaneous vasoconstriction.

Subsequently, there is an increased release of histamines and serotonins by the damaged cells. The resulting recurrence of vasodilation with a simultaneous increase in vascular permeability leads to the formation of wound oedema. This can be seen by the reddening and heating of the skin as well the resulting pain the patient experiences. In a later part of the phase, the cleansing of the wound is of primary importance. Oedematous fluid is exuded from the affected area in the form of protein rich exudate, cleaning the wound. At the same time, leukocyte infiltration in the affected area is stimulated. This last aspect results as well due to mechanical cleansing of germs and excess tissue, supported by biochemical processes for defence against germs and the active removal of irreversibly damaged tissues. The exudative phase serves to prepare the affected area for the following phases of the healing process. This particularly applies to the processes for chronic wounds, which do not always exhibit the bleeding mentioned at the beginning.

### 2. Granulation Phase

If the wound exhibits the corresponding prepared areas resulting from the exudative phase, then within the subsequent two to four days fibroblasts begin to collect and stroma is formed, also known as granulation tissue. The regeneration of tissue develops along the fibrin matrix of the blood coagulation and uses the supply of nutrients resulting from the simultaneous regeneration of blood vessels (angiogenesis).

### 3. Epithelization Phase

Through the initiation of contraction of the edges of the wound, the amount of necessary regeneration of tissue is reduced and epithelization phase begins. At this point, a regeneration of tissue begins at the edges of the wound, whereby keratinocytes or basal cells may be involved.

### 4. Reparative Phase

The epithelization is completed in the reparative phase, provided a corresponding prepared wound surface exists. The granulation tissue is converted to scar tissue. At this point the vessels recede, and over the course of months or even years, a hard, fibrous scar tissue forms. In this phase, proteases (particularly matrix metalloproteinases) may have particularly harmful effects.

The wound has different requirements during each phase, which are met by the wound care article of the invention as described in the following table:

| **Phase** | **Requirements for treatment and/or care** | **Corresponding active agent/principle as proved by the wound care article of the invention** |
|---|---|---|
| 1 | 1. Absorption of Exudate | Upper liquid absorbing layer (pref. with Super-absorbent particles) |
| | 2. Inhibition of bacteria | Bacteria adsorbing layer |
| | 3.Disinfection of the wound | Bacteria adsorbing layer: removal of bacterial colonization with each dressing change. |
| | 4. Inhibition of proteases | Bottom collagen(-like) layer |
| 2 | 1. Nourishing of the wound | peptide fragments from the bottom layer |
| | 2. Generation of a moist wound environment | Upper liquid absorbing layer (pref. with Super-absorbent particles) |
| | 3. Cell re-growth | Bottom layer provides scaffold for cell ingrowth / re-growth. |
| | 4. Formation of new extracellular matrix. | peptide fragments from the bottom layer promote deposition of extracellular matrix and provide substract for matrix building. |
| 3 | 1. Nourishing of the wound | peptide fragments from the bottom layer |
| | 2. Generation of a moist wound environment | Upper liquid absorbing layer (pref. with Super-absorbent particles) |
| | 3. Cell re-growth | Bottom layer provides scaffold for cell ingrowth / re-growth. |
| 4 | 1. Disinfection of the wound | Bacteria adsorbing layer: removal of bacterial colonization with each dressing change. |
| | 2. Protection of fragile tissue from shear stress and other mechanical irritation | Absorption and cushioning layer. |

### Negative pressure therapy

Also described herein is the use of the multi-layered wound care product according to the invention for negative pressure therapy. The usability in NPWT therapy is an important advantage given the various beneficial effects of this type of treatment. NPWT creates a moist environment, drains exudate, reduces tissue edema, contracts the wound edges, mechanically stimulates the wound bed, alters blood flow in the wound edges, and stimulates angiogenesis and the formation of granulation tissue. For said use it is preferred that the upper liquid absorbing layer of the multi-layered wound care product preferably comprises or consists of polyurethane foam.

In a third aspect the invention provides a negative pressure wound therapy (NPWT) treatment apparatus comprising;
(a) A multi-layered wound care product according the invention, sized and configured to be placed over and enclose a wound; wherein the dressing is adapted to maintain a reduced pressure between the dressing and the wound;
(b) A suction source configured to apply a reduced pressure to the wound care article; and
(c) Optionally a fluid collection device configured to remove and collect fluid and exudate aspirated from the wound by the suction source.

For the proper treatment of the wound it is necessary that the wound care product when placed over the wound encloses the wound.

Preferentially, the wound care article is preformed so as to correspond to the anatomic relief of a certain body position. This may be useful, for example, when the wound care article is to be applied to the elbow, hip, or knee areas. A deep-drawing process, for example, allows for the pre-forming of the wound care article so as to correspond to the anatomic relief of the aforementioned body positions.

Also preferentially, the wound care article is embodied such that it can adjust to the movements of a given body position. For these purposes, for example, the care article and the gas-tight closure for maintaining the reduced pressure may be embodied in an elastic manner. Furthermore, a pleated arrangement or one or several stretch bellows may be provided.

In the aforementioned embodiments it is particularly important that the aforementioned gas-tight closure will retain its gas-tightness property even under these aggravated conditions.

In a preferred embodiment of the invention the wound care article is attached to the skin of a patient by means of an adhesive material. For these purposes, every type of physiologically acceptable adhesive can be used, in particular medical-grade adhesives. Particularly preferred are materials selected from the group containing acrylic adhesives, silicone, hydrocolloid adhesives, zinc-oxide adhesives, and/or latex adhesives.

Hydrocolloid adhesives generally consist of a thin polymer film that is applied to a self-adhesive substance. The carrier substance (such as synthetic rubber types, for example poly-isobutylene) contains swelling particles, which vary, depending on the manufacturer. Often, swelling particles such as carboxymethyl cellulose or sodium carboxymethyl cellulose are included. Furthermore, they are very malleable, especially when warm. Hydrocolloid adhesives are suitable for being worked into surfaces, and are specifically capable of removing moisture. They are available in paste form, but also panel or strip form.

Something similar applies to silicone materials. The degree of adhesiveness to the skin can be regulated with these materials, so that despite the adhesiveness, a non-traumatic replacement of wound dressings can be ensured.

Preferentially, such silicone adhesives can be embodied in the form of a detachable self-adhesive laminate, which comprises a structural layer, with a wound-facing side to which a hydrophobic gel is applied, for example in the form of a silicone gels, and a side facing away from the wound, which carries an adhesive for example in the form of an acrylic adhesive.

Preferentially, said adhesive material is embodied in the form of a "border dressing" as an adhesive edge, which peripherally surrounds the wound-covering element.

Said adhesive material may also be embodied in the form of a panel or a strip on which the wound-covering element is distally positioned. In this embodiment, said panel or strip may feature a central opening, which is intended to be positioned over the wound. In that embodiment, said panel or strip takes the shape of a frame. Alternatively, said panel or strip may be embodied such that a window may be cut into the panel or the strip, corresponding in shape to the outline of the wound. For these purposes, the outline of the wound may be drawn on it, and then cut out with a pair of scissors. Alternatively, a template may be used, by means of which the outline of the wound can be transferred to the panel or the strip, or by means of which [an opening corresponding to] the outline of the wound can be cut out of the panel or the strip.

Said panel or said frame consists, for example of a hydrocolloid material as described herein. Said strip consists, for example, of a so-called incision foil, which is a self-adhesive foil made out of a polymer material.

Alternatively, said panel or said frame consists of a foam material and/or a spacer fabric. Preferentially, it is worked into a gas-tight cover. On the skin-facing side, the aforementioned adhesives may be applied.

In order to apply the reduced pressure to the wound care article the suction source is connected to the wound care device. For said purpose the suction source may be connected by the use of a coupling (such as in the Luer-Lock system) which allows an easy and reversible connection of a hose and/or a suction source.

The NPWT-treatment apparatus may further comprise an additional valve, a pressure reducer or even a window that can be opened. These make it possible for the device to be used even with an excessively strong negative pressure, for example when connected to a vacuum wall port, which can be frequently found in hospitals. Said valve, pressure reducer or window can then be opened in order to reduce the negative pressure.

In another embodiment, a liquid-impermeable barrier is located in the region of the connection device for ensuring that no fluids enter into the pump. Preferentially, said barrier comprises a semi-permeable membrane, for example one made out of a material such as Goretex, etc.

According to the invention, the NPWT-treatment apparatus also comprises a suction source for the generation of atmospheric negative pressure.

Said suction source or the generation of atmospheric negative pressure is preferentially selected from the group comprising: a) electrically operated vacuum pump; b) manually operated negative pressure source, and/or c) evacuated vacuum vessel.

The vacuum pump may be a solitary pump, but it may also be part of a centralized vacuuming system as often used in clinics. Hospital rooms are often fitted with vacuum wall ports to which the invented drainage devices for wound treatment can be connected. In this case, said vacuum pump can apply negative pressure to a plurality of drainage devices for wound treatment according to the invention.

Preferentially this is a micro-pump of which the dimensions and/or weight are such that they can be applied without difficulty to a wound-covering element of the aforementioned type, without bothering the patient or hindering him in any way.

For example, this pump may be a piezo- or membrane pump. Preferential in particular are piezo-pumps, which are pumps whose pumping capacity is brought about by a piezoelectrical element. These pumps have a sufficiently high pumping capacity despite its small dimensions. They further have low operating noise and low energy consumption. Alternatively, the pumps may also be a microsystems technology propellant-driven vacuum pump. Suitable pumps of this type are manufactured, for example, by Schwarzer Precision GmbH & Co. KG (Essen, Germany), KNF Neuberger GmbH (Freiburg, Germany), or Bartels Mikrotechnik GmbH (Dortmund, Germany).

Preferentially, such a pump is equipped with a check valve, which allows for the pump to be operated in interval mode, or only for the initial generation of negative pressure, or only in order to maintain negative pressure, without the occurrence of leaks during the operating pauses which would reduce the built-up negative pressure again.

In a preferred embodiment, said pump is capable of generating negative pressures of -60 to -200 mm Hg. Hereby 1 mm Hg equals 133,32 Pa. Preferentially in particular, the pump is capable of generating negative pressures of -60, -70, -80, -85, -90, -100, -110, -120, -130, -140, -150, -160, -170, -180, - 190 or -200 mm Hg.

Preferentially, the pump is selected or equipped such that it is capable of transporting fluids.

Preferentially, the operating noise of the pump does not exceed the acoustic pressure level of 65 dB. In particular, the acoustic pressure level of 63 dB, 60 dB, 58 dB, 55 dB, 53 dB, 50 dB, 48 dB, 45 dB, 42 dB, 40 dB, 38 dB, 35 dB, 32 dB, 30 dB, 28 dB, 25 dB, 22 dB, or even 20 dB is not exceeded.

Preferentially, the pump features a transportation rate of between 0.5 ml min⁻¹ and 100 ml min⁻¹. Preferentially, the transportation rate is between 2 ml min⁻¹ and 50 ml min⁻¹.

Most preferred transportation rates are between 10 ml min⁻¹ and 20 ml min⁻¹.

The aforementioned evacuated vessel can be connected to the wound care article of the invention for NPTW treatment in a manner similar to that of the previously known Redon bottle, and so apply negative pressure to it. Said evacuated vessel comprises an insert containing a fluid-absorbing polymer, preferentially in the form of a lining.

In a further preferred embodiment, said evacuated vessel can be embodied in the form of a cartridge, which is positioned in a mount which is already connected with the wound care article for NPWT treatment. When the cartridge is full, it can be removed and disposed of, and a new evacuated cartridge can be placed in the mount.

The aforementioned embodiments are particularly advantageous, because they dispense with the need for a pump of their own, and instead use an evacuated vessel which makes the treatment apparatus mobile and independent of the power grid, with as a result that the patient himself becomes mobile. Moreover, this allows for a smaller construction which allows the patient to discretely conceal the apparatus. Particularly advantageous in this respect is an anatomically adjusted embodiment of said evacuated vessel or mount, which allows for an inconspicuous carrying thereof, for instance on the leg.

Moreover, such a treatment apparat produces no operating noises, and is very easy to operate.

The same applies to the aforementioned manually operated negative pressure source. In the simplest embodiment, this may be a plastic syringe with a sufficiently high volume. Other options are a pump shaped as a rubber ball, bellows, etc.

In a further preferred embodiment, the facility for the generation of atmospheric negative pressure is positioned directly on the wound care article.

This may be accomplished, for example, by positioning said facility directly onto the wound care article. In this way, a separate vacuum hose, which involves manufacturing challenges (sufficient rigidity in order not to collapse under vacuum conditions) as well as hygienic problems (risk of contamination) can be eliminated, or be kept very short.

In another embodiment, a coupling, a blocking valve, and/or a three-way valve is positioned between the facility for the generation of atmospheric negative pressure and the wound care article or the fluid collection device.

This device allows for ensuring that a) the facility for the generation of atmospheric negative pressure can be disconnected from the test of the wound care article, b) a once generated negative pressure can be retained for the longest possible time, and/or c) in order to spare the battery or when the battery is empty, an external pump or an external vacuum vessel can be used to generate or restore negative pressure.

Said external pump or said external vacuum vessel may be stationary in the clinic or in the patient's home, for example, but it may also be embodied in a mobile form (for example in the form of a suitcase, or integrated in the patient's clothing, or attached to the patient's belt). This is to ensure that the device on the wound remains small and inconspicuous, since its primary task is to maintain the vacuum, but to provide sufficient pumping capacity when needed in order to allow for an efficient therapy.

### Preparation method

In a fourth aspect the invention provides a method for preparation of the multi-layered wound care product of the invention comprising the following steps:
(a) A liquid absorbing material (such as a superabsorbent) is ultrasonically sealed into a non-woven material;
(b) The bacteria absorbing layer is bonded to one side of the non-woven material by using a hotmelt;
(c) Using an intermediate layer of silicone-based glue, the liquid-permeable bottom layer is attached onto the bacteria absorbing layer.

Also disclosed herein is a method for preparation of a multi-layered wound care product comprising the following steps:
(a) During the production of the liquid-permeable bottom layer, the bacteria-adsorbing layer is included directly into the manufacturing process, thus yielding a blended material;
(b) A liquid absorbing material (such as a superabsorbent) is ultrasonically sealed into a non-woven material;
(c) The two layers of step (a) and step (b) are joined together by use of a hotmelt or by ultrasound sealing.

Further disclosed herein is a method for preparation of a multi-layered wound care product comprising the following steps:
(a) A thin polyurethane film material is coated on the whole surface with an acrylic adhesive.
(b) A liquid-absorbing material (such as polyurethane foam) is directly attached centered onto this adhesive layer, thereby leaving a sufficiently large peripheral border of the adhesive film material.
(c) Using a hotmelt, the bacteria-adsorbing layer is attached onto the liquid-absorbing material, while both materials having the same dimensions.
(d) The liquid-permeable bottom layer - having greater dimensions than the bacteria-adsorbing layer and the liquid-absorbing material - is directly attached centered onto the adhesive polyurethane film material (top layer), while leaving a sufficiently large border of the adhesive film material in order to be attached onto e.g. patient skin. The bacteria-adsorbing layer and the liquid-absorbing material will then be partly covered by the liquid-permeable bottom layer.

### Brief description of the drawings:

These and other aspects of the invention will be apparent from and elucidated with reference to the embodiments described hereinafter.

The invention will now be described, by way of example, based on embodiments with reference to the accompanying drawings.

In the drawings:
Fig. 1 shows a principal sketch of the wound care product according to a first embodiment in cross section (A) or in a perspective view (B).
Fig. 2 shows schematically sketches of the wound care product according to further embodiments in cross section with three different layer constitutions in (A) to (C).
Fig. 3 shows a principal sketches of the wound care product according to further embodiments in front view with six different types of incisions or perforations.
Fig. 4 shows principal sketches of the wound care product according to further embodiments in front view with five different types of incisions or perforations.

In the Figures, like numbers refer to like objects throughout. Objects in the Figures are not necessarily drawn to scale.

### Detailed description of embodiments:

Various embodiments of the invention will now be described by means of the Figures.

Fig. 1 shows a principal sketch of the wound care product according to a first embodiment in cross section (A) or in a perspective view (B) consisting of three layers, namely the liquid-permeable bottom layer (LP), the intermediate bacteria adsorbing layer (BA) and the liquid-absorbing layer (LA) as top layer. The wound care product is shown in a cross section/perspective view and may be applied as such to a skin wound. To this, the wound care product has a surface that will face the wound when being applied and will come in direct contact with the wound, and has an opposite surface that will face away from the wound and will thus initially not contact the wound when applied. '

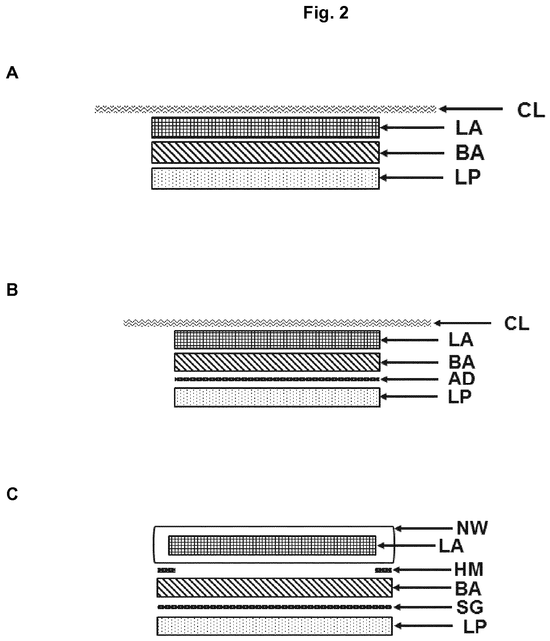

Fig. 2 shows further embodiments of the wound care product as follows:
In (A) a wound care product with a liquid-permeable bottom layer (LP), an intermediate bacteria adsorbing layer (BA) and a liquid-absorbing layer (LA) as top layer is shown whereby the LA layer is covered by a cover layer (CA) protruding said three layers.
In (B), the wound care product according Fig. 2A, whereby the bottom layer LP and the intermediate layer BA are connected by a layer AD of a biodegradable adhesive.
In (C) a wound care product comprising a liquid-permeable bottom layer (LP), an intermediate bacteria adsorbing layer (BA) and a liquid-absorbing layer (LA) as top layer, whereby the liquid-absorbing layer LA is embedded in a layer NW of non-woven material. Furthermore the layer NW and the bacteria-adsorbing layer are connected in the periphery by a hot melt adhesive HM. Furthermore, the bottom layer LP and the intermediate layer BA are connected by an layer SG of an silicone-based glue.

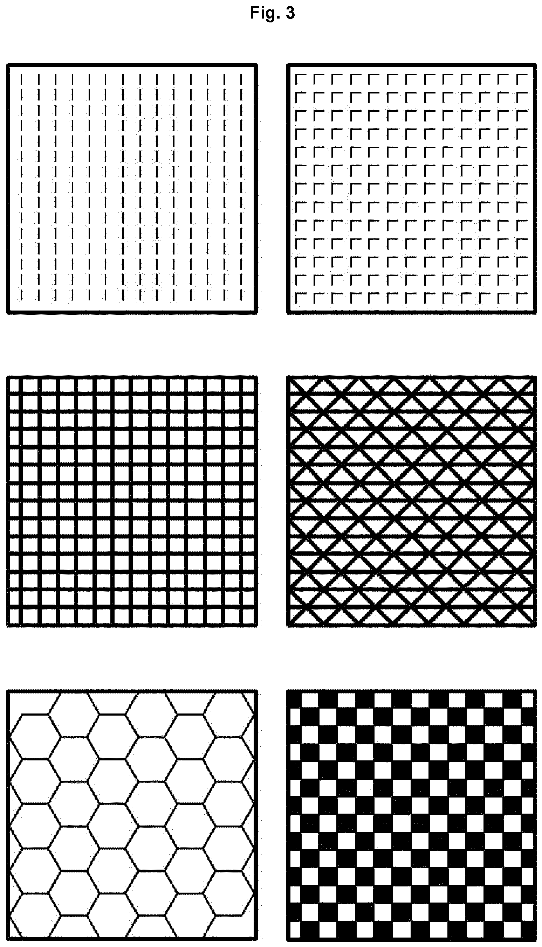

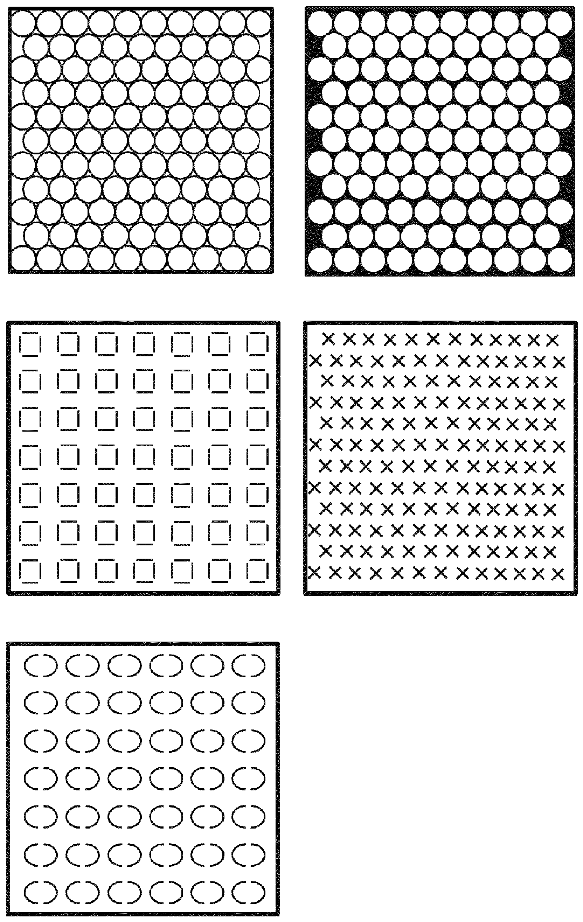

Figure 3 shows six different embodiments for perforations of the liquid-permeable bottom of the wound care product. A front view of the wound care article shows from upper left to lower right: rows of linear incisions in black; a regular pattern of angular incisions in black, quadratic recesses in white leaving a rectangular grid of the bottom layer presented in black; a regular pattern of triangular recesses in white leaving a triangular grid of the bottom layer in black; a regular pattern of hexangular recesses in white leaving a hexangular, honeycomb-like grid of the bottom layer presented in black, a regular pattern of quadratic recesses in white leaving a quadratic chess board-like grid of the bottom layer in black.

Figure 4 shows five different embodiments for perforations of the liquid-permeable bottom of the wound care product. A front view of the wound care article shows from upper left to lower left: a bottom layer build from neighboring circular rings in black, whereby the underlying intermediate layer is given in white; a regular pattern of circular recesses in white; a regular pattern of squares formed by four incisions each in black, a regular pattern of cross-like incisions in black; a regular pattern of ellipsoids formed by two incisions each in black.

While the invention has been illustrated and described in detail in the drawings and the foregoing description, such illustration and description are to be considered illustrative or exemplary and not restrictive.

From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

### List of reference signs:

- 1: LA: liquid absorbing layer
- 2: BA: bacteria adsorbing layer
- 3: LP: liquid permeable bottom layer
- 4: CL: cover layer
- 5: AD: biodegradable adhesive
- 6: NW: non-woven material
- 7: HM: Hotmelt
- 8: SG: silicone-based glue

## Claims

1. A multilayered wound care product comprising:
(a) an upper liquid-absorbing layer;
(b) an intermediate bacteria-adsorbing layer; and
(c) a liquid-permeable bottom layer comprising collagen, a recombinant collagen-like protein of bacterial origin which comprises at least one MMP cleavage site or peptides comprising a matrix metalloprotease (MMP) cleavage site or a combination thereof; and optionally
(d) a cover layer situated above the upper liquid-absorbing layer;
wherein the liquid-permeable bottom layer is a continuous sheet-like layer having perforations or incisions and wherein the liquid permeability of said bottom layer is given by said perforations or incisions enabling the passage of liquid through said layer,
and wherein the collagen-like protein comprises the formula III:
(III) [(Gly-Xaa-Yaa)ₘ-(insert)ₙ]ₚ
where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site and wherein the overall content of Xaa and Yaa provides a proline rich structure where the total percentage of proline of all residues in the Xaa and Yaa positions is greater than 19%.

2. A multilayered wound care product comprising:
(a) an upper liquid-absorbing layer comprising a polymeric foam, wherein the foam is a polyurethane foam;
(b) a liquid-permeable bottom layer comprising collagen, a recombinant collagen-like protein of bacterial origin which comprises at least one MMP cleavage site or peptides comprising a matrix metalloprotease (MMP) cleavage site or a combination thereof; and optionally
(d) a cover layer situated above the upper liquid-absorbing layer
wherein the liquid-permeable bottom layer is a continuous sheet-like layer having incisions and wherein the liquid permeability of said bottom layer is given by said incisions enabling the passage of liquid through said layer and wherein the collagen-like protein comprises the formula III:
(III) [(Gly-Xaa-Yaa)ₘ-(insert)ₙ]ₚ
where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site and wherein the overall content of Xaa and Yaa provides a proline rich structure where the total percentage of proline of all residues in the Xaa and Yaa positions is greater than 19%,
and wherein the protein collagen is not included in the upper liquid absorbing layer, and wherein the different layers are connected by the use of an adhesive.

3. The multilayered wound care product according claim 1, wherein the upper liquid-absorbing layer contains at least one absorbent material selected from the group consisting of polymer foams, sponges, hydrocolloids, hydrogels and hydrophilic polymers such as superabsorbent polymers.

4. The multilayered wound care product according claims 1 or 3, wherein the intermediate bacteria-adsorbing layer comprises a hydrophobic or hydrophobized carrier material, which is preferably a hydrophilic carrier material treated with silicone, dialkyl carbamoyl chloride and/or alkyl ketene dimer (AKD).

5. The multi-layered wound care product according to claims 1, 3 or 4, wherein the intermediate bacteria-adsorbing layer is a cellulose layer, being preferably a cellulose acetate layer, coated with dialkyl carbamoyl chloride.

6. The multi-layered wound care product according to any of the above claims, wherein the upper liquid-absorbing layer and/or the intermediate bacteria-adsorbing layer further comprises at least one antimicrobial active compound, preferably selected from the group consisting of bacteriocin like inhibitory substance (BLIS), silver based compound, biguanide salt like polyhexamethylbiguanide (PHMB), chlorhexidine.

7. The multi-layered wound care product according to claims 1 or 3 to 6, wherein the perforations of the continuous sheet-like liquid-permeable bottom layer allow a direct contact of the intermediate layer with the wound site.

8. The multi-layered wound care product according to any of the above claims, wherein the liquid-permeable bottom layer has a thickness between 0.5 and 3 mm, preferably between 0.8 and 1.8 mm, and more preferably between 1.0 and 1.5 mm.

9. The multi-layered wound care product according to any of the above claims, wherein the liquid-permeable bottom layer is a grid-like structure with trigonal, tetragonal or hexagonal meshes.

10. The multi-layered wound care product according to claim 7, wherein the perforated bottom liquid-permeable layer allows a direct contact of the overlaying layer, being preferably the bacteria-adsorbing layer, with the wound site.

11. The multi-layered wound care product according to claim 7 to 10, wherein the perforations account for between 20 to 80%, preferably between 30 to 70% and most preferably between 40 and 60% of the area of the liquid-permeable bottom layer.

12. The multi-layered wound care product according to any of the above claims, wherein the collagen of the bottom liquid-permeable layer is human recombinant collagen or type I, II or III collagen obtained from one of the following sources: type I collagen: bovine, chicken and fish skin, bovine and chicken tendons and bovine and chicken bones including fetal tissues; type II collagen: bovine articular cartilage, nasal septum, sternal cartilage; and type III collagen: bovine and human aorta and skin.

13. The multi-layered wound care product according to any of the above claims, wherein the cover layer is a water-impervious vapor-permeable membrane, preferably comprising a polymer material selected from the group consisting of polyurethane, polyester, polyvinyl chloride or mixtures thereof.

14. The multi-layered wound care product according to any of the above claims, wherein the an upper liquid-absorbing layer, the intermediate bacteria-adsorbing layer, and a liquid-permeable bottom layer are bonded at the border portion or at the edges of said layers, whereby the layers are adhered to the respective neighboring layer, to the upper cover layer or to a further outermost non-woven layer as wound contacting layer.

15. The multi-layered wound-care product according to any of the above claims for use in the treatment of chronic wounds, comprising burns, partial and full-thickness wounds, pressure ulcers, venous ulcers, arterial ulcers, diabetic ulcers, chronic vascular ulcers, draining wounds, tunnelled or undermined wounds, surgical wounds, post-Mohs surgery, post-laser surgery, podiatric dehiscence, and wound dehiscence, trauma wounds, lacerations, second-degree burns, and skin tears.

16. Multi-layered wound care product according to any of the above claims for use in negative pressure therapy, wherein the upper liquid absorbing layer of the multi-layered wound care product comprises or consists of polyurethane foam.

17. A negative pressure therapy wound treatment apparatus comprising;
(a) A multi-layered wound care product according to any of claims 1 to 15, sized and configured to be placed over and enclose a wound; wherein the dressing is adapted to maintain a reduced pressure between the dressing and the wound;
(b) A suction source configured to apply a reduced pressure to the wound care article; and
(c) Optionally a fluid collection device configured to remove and collect fluid and exudate aspirated from the wound by the suction source.

18. A method of preparing a multilayered wound care product according to any of claim 1 or claims 3 to 11 comprising the steps of:
(a) A liquid absorbing material (such as a superabsorbent) is ultrasonically sealed into a non-woven material;
(b) The bacteria absorbing layer is bonded to one side of the non-woven material by using a hotmelt;
(c) Using an intermediate layer of silicone-based glue, the liquid-permeable bottom layer is attached onto the bacteria absorbing layer.

## Patentansprüche

1. Mehrschichtiges Wundpflegeprodukt, umfassend:
(a) eine obere flüssigkeitsabsorbierende Schicht;
(b) eine bakterienadsorbierende Zwischenschicht; und
(c) eine flüssigkeitsdurchlässige untere Schicht, umfassend Kollagen, ein rekombinantes kollagenähnliches Protein bakteriellen Ursprungs, das mindestens eine MMP-Spaltstelle umfasst, oder Peptide, die eine Matrix-Metalloprotease-Spaltstelle (MMP-Spaltstelle) umfassen, oder eine Kombination davon; und optional
(d) eine Deckschicht, die sich über der oberen flüssigkeitsabsorbierenden Schicht befindet;
wobei die flüssigkeitsdurchlässige untere Schicht eine kontinuierliche blattartige Schicht mit Perforationen oder Einschnitten ist und wobei die Flüssigkeitsdurchlässigkeit der unteren Schicht durch die Perforationen oder Einschnitte gegeben ist, die den Durchgang von Flüssigkeit durch die Schicht ermöglichen,
und wobei das kollagenähnliche Protein die Formel III umfasst:
(III) [(Gly-Xaa-Yaa)ₘ-(Insert)ₙ]ₚ
wobei m zwischen 1 und 200 liegt und (Gly-Xaa-Yaa)ₘ eine dreifach helikale Domäne darstellt, wobei Xaa und Yaa unabhängig voneinander beliebige natürliche und unnatürliche Imino- oder Aminosäuren für jede Wiederholungseinheit sind, wobei das Insert aus 1 bis 50 beliebigen Imino- oder Aminosäuren besteht, wobei n 0 oder 1 ist und p eine beliebige Zahl zwischen 2 und 10 ist, wobei der Wert von n für jede Wiederholung eindeutig ist und mindestens ein Insert im kollagenähnlichen Protein vorhanden ist, das mindestens eine MMP-Spaltstelle umfasst, und wobei der Gesamtgehalt an Xaa und Yaa eine prolinreiche Struktur bereitstellt, bei der der Gesamtprozentsatz an Prolin aller Reste in den Positionen Xaa und Yaa größer als 19 % ist.

2. Mehrschichtiges Wundpflegeprodukt, umfassend:
(a) eine obere flüssigkeitsabsorbierende Schicht, die einen Polymerschaum umfasst, wobei der Schaum ein Polyurethanschaum ist;
(b) eine flüssigkeitsdurchlässige untere Schicht, umfassend Kollagen, ein rekombinantes kollagenähnliches Protein bakteriellen Ursprungs, das mindestens eine MMP-Spaltstelle umfasst, oder Peptide, die eine Matrix-Metalloprotease-Spaltstelle (MMP-Spaltstelle) umfassen, oder eine Kombination davon; und optional
(d) eine Deckschicht, die sich über der oberen flüssigkeitsabsorbierenden Schicht befindet,
wobei die flüssigkeitsdurchlässige untere Schicht eine kontinuierliche blattartige Schicht mit Einschnitten ist und wobei die Flüssigkeitsdurchlässigkeit der unteren Schicht durch die Einschnitte gegeben ist, die den Durchgang von Flüssigkeit durch die Schicht ermöglichen, und wobei das kollagenartige Protein die Formel III umfasst:
(III) [(Gly-Xaa-Yaa)ₘ-(Insert)ₙ]ₚ
wobei m zwischen 1 und 200 liegt und (Gly-Xaa-Yaa)ₘ eine dreifach helikale Domäne darstellt, wobei Xaa und Yaa unabhängig voneinander jede natürliche und unnatürliche Imino- oder Aminosäure für jede Wiederholungseinheit sind, wobei das Insert aus 1 bis 50 beliebigen Imino- oder Aminosäuren besteht, wobei n 0 oder 1 ist und p eine beliebige Zahl zwischen 2 und 10 ist, wobei der Wert von n für jede Wiederholung eindeutig ist und mindestens ein Insert im kollagenähnlichen Protein vorhanden ist, das mindestens eine MMP-Spaltstelle umfasst, und wobei der Gesamtgehalt an Xaa und Yaa eine prolinreiche Struktur bereitstellt, bei der der Gesamtprozentsatz an Prolin aller Reste in den Positionen Xaa und Yaa größer als 19 % ist,
und wobei das Proteinkollagen nicht in der oberen flüssigkeitsabsorbierenden Schicht enthalten ist,
und wobei die verschiedenen Schichten durch die Verwendung eines Klebstoffs verbunden sind.

3. Mehrschichtiges Wundpflegeprodukt gemäß Anspruch 1, wobei die obere flüssigkeitsabsorbierende Schicht mindestens ein absorbierendes Material enthält, das aus der Gruppe ausgewählt ist, die aus Polymerschäumen, Schwämmen, Hydrokolloiden, Hydrogelen und hydrophilen Polymeren wie superabsorbierenden Polymeren besteht.

4. Mehrschichtiges Wundpflegeprodukt gemäß Anspruch 1 oder 3, wobei die bakterienadsorbierende Zwischenschicht ein hydrophobes oder hydrophobiertes Trägermaterial umfasst, das vorzugsweise ein hydrophiles Trägermaterial ist, das mit Silikon, Dialkylcarbamoylchlorid und/oder Alkylketendimer (AKD) behandelt ist.

5. Mehrschichtiges Wundpflegeprodukt gemäß den Ansprüchen 1, 3 oder 4, wobei die bakterienadsorbierende Zwischenschicht eine Zelluloseschicht ist, vorzugsweise eine Zelluloseacetatschicht, die mit Dialkylcarbamoylchlorid beschichtet ist.

6. Mehrschichtiges Wundpflegeprodukt gemäß einem der vorstehenden Ansprüche, wobei die obere flüssigkeitsabsorbierende Schicht und/oder die bakterienadsorbierende Zwischenschicht ferner mindestens eine antimikrobiell aktive Verbindung umfasst, die vorzugsweise aus der Gruppe ausgewählt ist, die aus bakteriozinähnlicher Inhibitorsubstanz (BLIS), silberbasierter Verbindung, Biguanidsalz wie Polyhexamethylbiguanid (PHMB) und Chlorhexidin besteht.

7. Mehrschichtiges Wundpflegeprodukt gemäß den Ansprüchen 1 oder 3 bis 6, wobei die Perforationen der kontinuierlichen, blattartigen, flüssigkeitsdurchlässigen unteren Schicht einen direkten Kontakt der Zwischenschicht mit der Wundstelle ermöglichen.

8. Mehrschichtiges Wundpflegeprodukt gemäß einem der vorstehenden Ansprüche, wobei die flüssigkeitsdurchlässige untere Schicht eine Dicke zwischen 0,5 und 3 mm, vorzugsweise zwischen 0,8 und 1,8 mm und mehr bevorzugt zwischen 1,0 und 1,5 mm aufweist.

9. Mehrschichtiges Wundpflegeprodukt gemäß einem der vorstehenden Ansprüche, wobei die flüssigkeitsdurchlässige untere Schicht eine gitterartige Struktur mit trigonalen, tetragonalen oder hexagonalen Maschen ist.

10. Mehrschichtiges Wundpflegeprodukt gemäß Anspruch 7, wobei die perforierte flüssigkeitsdurchlässige untere Schicht einen direkten Kontakt der darüber liegenden Schicht, vorzugsweise der bakterienadsorbierenden Schicht, mit der Wundstelle ermöglicht.

11. Mehrschichtiges Wundpflegeprodukt gemäß Anspruch 7 bis 10, wobei die Perforationen zwischen 20 und 80 %, vorzugsweise zwischen 30 und 70 % und am meisten bevorzugt zwischen 40 und 60 % der Fläche der flüssigkeitsdurchlässigen unteren Schicht ausmachen.

12. Mehrschichtiges Wundpflegeprodukt gemäß einem der vorstehenden Ansprüche, wobei das Kollagen der unteren flüssigkeitsdurchlässigen Schicht menschliches rekombinantes Kollagen oder Kollagen vom Typ I, II oder III ist, das aus einer der folgenden Quellen gewonnen wird: Kollagen vom Typ I: Rinder-, Hühner- und Fischhaut, Rinder- und Hühnersehnen und Rinder- und Hühnerknochen einschließlich fötalem Gewebe; Kollagen vom Typ II: Gelenkknorpel vom Rind, Nasenscheidewand, Sternumknorpel; und Kollagen vom Typ III: Aorta und Haut von Rindern und Menschen.

13. Mehrschichtiges Wundpflegeprodukt gemäß einem der vorstehenden Ansprüche, wobei die Deckschicht eine wasserundurchlässige, dampfdurchlässige Membran ist, die vorzugsweise ein Polymermaterial umfasst, das aus der Gruppe ausgewählt ist, die aus Polyurethan, Polyester, Polyvinylchlorid oder Mischungen davon besteht.

14. Mehrschichtiges Wundpflegeprodukt gemäß einem der vorstehenden Ansprüche, wobei eine obere flüssigkeitsabsorbierende Schicht, die bakterienadsorbierende Zwischenschicht und eine flüssigkeitsdurchlässige untere Schicht an den Randbereichen oder an den Kanten der genannten Schichten verbunden sind, wobei die Schichten mit der jeweils benachbarten Schicht, der oberen Deckschicht oder einer weiteren äußersten Vliesschicht als Wundkontaktschicht verklebt sind.

15. Mehrschichtiges Wundpflegeprodukt gemäß einem der vorstehenden Ansprüche zur Verwendung bei der Behandlung von chronischen Wunden, umfassend Verbrennungen, Wunden teilweiser und vollständiger Dicke, Druckgeschwüre, venöse Geschwüre, arterielle Geschwüre, diabetische Geschwüre, chronische Gefäßgeschwüre, nässende Wunden, tunnelartige oder unterminierte Wunden, chirurgische Wunden, Wunden nach Mohs-Chirurgie, nach Laserchirurgie, podologischer Dehiszenz und Wunddehiszenz, Traumawunden, Schnittwunden, Verbrennungen zweiten Grades und Hautrisse.

16. Mehrschichtiges Wundpflegeprodukt nach einem der vorstehenden Ansprüche zur Verwendung in der Unterdrucktherapie, wobei die obere flüssigkeitsabsorbierende Schicht des mehrschichtigen Wundpflegeprodukts Polyurethanschaum umfasst oder daraus besteht.

17. Gerät zur Wundbehandlung mit Unterdrucktherapie, umfassend:
(a) ein mehrschichtiges Wundpflegeprodukt nach einem der Ansprüche 1 bis 15, das so bemessen und beschaffen ist, dass es über eine Wunde gelegt werden kann und diese umschließt; wobei der Wundverband dazu geeignet ist, einen reduzierten Druck zwischen dem Wundverband und der Wunde aufrechtzuerhalten;
(b) eine Saugquelle, die konfiguriert ist, einen Unterdruck auf den Wundpflegeartikel auszuüben; und
(c) optional eine Flüssigkeitssammelvorrichtung, die konfiguriert ist, um Flüssigkeit und Exsudat, die von der Saugquelle aus der Wunde abgesaugt werden, zu entfernen und zu sammeln.

18. Verfahren zur Herstellung eines mehrschichtigen Wundpflegeprodukts gemäß einem der Ansprüche 1 oder 3 bis 11, umfassend die folgenden Schritte:
(a) ein flüssigkeitsabsorbierendes Material (wie etwa ein Superabsorber) wird mittels Ultraschall in ein Vliesmaterial versiegelt;
(b) die bakterienabsorbierende Schicht wird mittels eines Hotmelts auf eine Seite des Vliesmaterials geklebt;
(c) mittels einer Zwischenschicht aus silikonbasiertem Kleber wird die flüssigkeitsdurchlässige untere Schicht auf die bakterienabsorbierende Schicht angebracht.

## Revendications

1. Produit de soin de plaie multicouche comprenant :
(a) une couche supérieure absorbant les liquides ;
(b) une couche intermédiaire adsorbant les bactéries ; et
(c) une couche inférieure perméable aux liquides comprenant du collagène, une protéine recombinante de type collagène d'origine bactérienne qui comprend au moins un site de clivage de MMP ou des peptides comprenant un site de clivage de métalloprotéase matricielle (MMP) ou une combinaison de ceux-ci ; et facultativement
(d) une couche de recouvrement située au-dessus de la couche supérieure absorbant les liquides ;
dans lequel la couche inférieure perméable aux liquides est une couche continue en forme de feuille ayant des perforations ou des incisions et dans lequel la perméabilité aux liquides de ladite couche inférieure est donnée par lesdites perforations ou incisions qui permettent le passage de liquides à travers ladite couche,
et dans lequel la protéine de type collagène comprend la formule III :
(III) [(Gly-Xaa-Yaa)ₘ-(insert)ₙ]ₚ
où m est compris entre 1 et 200 et (Gly-Xaa-Yaa)ₘ représente un domaine hélicoïdal triple, Xaa et Yaa étant indépendamment tout imino ou acide aminé naturel ou non naturel pour chaque unité de répétition, où l'insert est composé de 1 à 50 de tout imino ou acide aminé, n est 0 ou 1, et p est tout nombre compris entre 2 et 10, où la valeur de n est unique pour chaque répétition et au moins un insert est fourni dans la protéine de type collagène qui comprend au moins un site de clivage de MMP et où le contenu global de Xaa et Yaa fournit une structure riche en proline où le pourcentage total de proline de tous les résidus dans les positions Xaa et Yaa est supérieur à 19 %.

2. Produit de soin de plaie multicouche comprenant :
(a) une couche supérieure absorbant les liquides, comprenant une mousse polymère, dans lequel la mousse est une mousse de polyuréthane ;
(b) une couche inférieure perméable aux liquides comprenant du collagène, une protéine recombinante de type collagène d'origine bactérienne qui comprend au moins un site de clivage de MMP ou des peptides comprenant un site de clivage de métalloprotéase matricielle (MMP) ou une combinaison de ceux-ci ; et facultativement
(d) une couche de recouvrement située au-dessus de la couche supérieure absorbant les liquides
dans lequel la couche inférieure perméable aux liquides est une couche continue en forme de feuille ayant des incisions et dans lequel la perméabilité aux liquides de ladite couche inférieure est donnée par lesdites incisions permettant le passage de liquides à travers ladite couche et dans lequel la protéine de type collagène comprend la formule III :
(III) [(Gly-Xaa-Yaa)ₘ-(insert)ₙ]ₚ
où m est compris entre 1 et 200 et (Gly-Xaa-Yaa)ₘ représente un domaine hélicoïdal triple, Xaa et Yaa étant indépendamment tout imino ou acide aminé naturel ou non naturel pour chaque unité de répétition, où l'insert est composé de 1 à 50 de tout imino ou acide aminé, n est 0 ou 1, et p est tout nombre compris entre 2 et 10, où la valeur de n est unique pour chaque répétition et au moins un insert est fourni dans la protéine de type collagène qui comprend au moins un site de clivage de MMP et où le contenu global de Xaa et Yaa fournit une structure riche en proline où le pourcentage total de proline de tous les résidus dans les positions Xaa et Yaa est supérieur à 19 %,
et dans lequel la protéine de collagène n'est pas incluse dans la couche supérieure absorbant les liquides,
et dans lequel les différentes couches sont reliées par l'utilisation d'un adhésif.

3. Produit de soin de plaie multicouche selon la revendication 1, dans lequel la couche supérieure absorbant les liquides contient au moins un matériau absorbant choisi dans le groupe constitué de mousses polymères, éponges, hydrocolloïdes, hydrogels et polymères hydrophiles tels que des polymères superabsorbants.

4. Produit de soin de plaie multicouche selon les revendications 1 ou 3, dans lequel la couche intermédiaire adsorbant les bactéries comprend un support hydrophobe ou hydrophobisé, qui est de préférence un support hydrophile traité avec de la silicone, du chlorure de dialkyle carbamoyle et/ou du dimère d'alkylcétène (AKD).

5. Produit de soin de plaie multicouche selon les revendications 1, 3 ou 4, dans lequel la couche intermédiaire adsorbant les bactéries est une couche de cellulose, de préférence une couche d'acétate de cellulose, recouverte de chlorure de dialkyle carbamoyle.

6. Produit de soin de plaie multicouche selon l'une quelconque des revendications précédentes, dans lequel la couche supérieure absorbant les liquides et/ou la couche intermédiaire adsorbant les bactéries comprend en outre au moins un composé actif antimicrobien, de préférence choisi dans le groupe constitué de substance inhibitrice de type bactériocine (BLIS), de composé à base d'argent, de sel de biguanide tel que le polyhexaméthylbiguanide (PHMB), de chlorhexidine.

7. Produit de soin de plaie multicouche selon les revendications 1 ou 3 à 6, dans lequel les perforations de la couche inférieure continue perméable aux liquides permettent un contact direct de la couche intermédiaire avec le site de plaie.

8. Produit de soin de plaie multicouche selon l'une quelconque des revendications précédentes, dans lequel la couche inférieure perméable aux liquides a une épaisseur comprise entre 0,5 et 3 mm, de préférence entre 0,8 et 1,8 mm, et plus préférablement entre 1,0 et 1,5 mm.

9. Produit de soin de plaie multicouche selon l'une quelconque des revendications précédentes, dans lequel la couche inférieure perméable aux liquides est une structure en forme de grille avec des mailles trigonales, tétragonales ou hexagonales.

10. Produit de soin de plaie multicouche selon la revendication 7, dans lequel la couche inférieure perforée perméable aux liquides permet un contact direct de la couche de recouvrement, qui est de préférence la couche adsorbant les bactéries, avec le site de plaie.

11. Produit de soin de plaie multicouche selon les revendications 7 à 10, dans lequel les perforations représentent entre 20 et 80 %, de préférence entre 30 et 70 % et plus préférablement entre 40 et 60 % de la surface de la couche inférieure perméable aux liquides.

12. Produit de soin de plaie multicouche selon l'une quelconque des revendications précédentes, dans lequel le collagène de la couche inférieure perméable aux liquides est du collagène recombinant humain ou du collagène de type I, II ou III obtenu à partir de l'une des sources suivantes : le collagène de type I : peau de bovin, de poulet et de poisson, tendons de bovin et de poulet et os de bovin et de poulet, y compris les tissus fœtaux ; le collagène de type II : cartilage articulaire bovin, septum nasal, cartilage sternal ; et le collagène de type III : aorte et peau de bovin et humaines.

13. Produit de soin de plaie multicouche selon l'une quelconque des revendications précédentes, dans lequel la couche de recouvrement est une membrane imperméable à l'eau et perméable à la vapeur, comprenant de préférence un matériau polymère choisi dans le groupe constitué de polyuréthane, polyester, chlorure de polyvinyle ou de mélanges de ceux-ci.

14. Produit de soin de plaie multicouche selon l'une quelconque des revendications précédentes, dans lequel la couche supérieure absorbant les liquides, la couche intermédiaire adsorbant les bactéries et la couche inférieure perméable aux liquides sont collées au niveau de la partie de bordure ou au niveau des bords desdites couches, moyennant quoi les couches adhèrent à la couche voisine respective, à la couche de recouvrement supérieure ou à une autre couche non tissée extérieure en tant que couche de contact avec la plaie.

15. Produit de soin de plaie multicouche selon l'une quelconque des revendications précédentes, destiné à être utilisé dans le traitement de plaies chroniques, comprenant des brûlures, des plaies partielles et profondes, des ulcères de pression, des ulcères veineux, des ulcères artériels, des ulcères diabétiques, des ulcères vasculaires chroniques, des plaies drainantes, des plaies tunnelisées ou sous-minées, des plaies chirurgicales, une chirurgie post-Mohs, une chirurgie post-laser, des déhiscences podiatriques, et des déhiscences de plaies, des plaies traumatiques, des lacérations, des brûlures du second degré et des déchirures cutanées.

16. Produit de soin de plaie multicouche selon l'une quelconque des revendications précédentes destiné à être utilisé en thérapie par pression négative, dans lequel la couche supérieure absorbant les liquides du produit de soin de plaie multicouche comprend ou est constitué de mousse de polyuréthane.

17. Appareil de traitement de plaie par pression négative comprenant :
(a) Un produit de soin de plaie multicouche selon l'une quelconque des revendications 1 à 15, dimensionné et conçu pour être placé sur une plaie et l'envelopper ; dans lequel le pansement est adapté pour maintenir une pression réduite entre le pansement et la plaie ;
(b) Une source d'aspiration conçue pour appliquer une pression réduite à l'article de soin de plaie ; et
(c) Facultativement, un dispositif de collecte des fluides conçu pour retirer et collecter les fluides et les exsudats aspirés depuis la plaie par la source d'aspiration.

18. Procédé de préparation d'un produit de soin de plaie multicouche selon l'une quelconque de la revendication 1 ou des revendications 3 à 11, comprenant les étapes suivantes :
(a) Un matériau absorbant les liquides (tel qu'un superabsorbant) est scellé par ultrasons dans un matériau non tissé ;
(b) La couche absorbant les bactéries est collée sur une face du matériau non tissé à l'aide d'un adhésif thermofusible ;
(c) L'utilisation d'une couche intermédiaire de colle à base de silicone, la couche inférieure perméable aux liquides est fixée sur la couche absorbant les bactéries.
